# EUROPEAN PATENT SPECIFICATION

(11) **EP 1 568 283 B1**
(45) Date of publication and mention of the grant of the patent: **26.08.2009**
(21) Application number: 03811909.5
(22) Date of filing: 20.11.2003
(51) Int. Cl.: A23L 1/015

(54) **METHOD OF INHIBITING ACRYLAMIDE FORMATION AND USE THEREOF**
VERFAHREN ZUR HEMMUNG DER ACRYLAMIDBILDUNGUND SEINE VERWENDUNG
PROCEDE PERMETTANT D'INHIBER LA FORMATION D'ACRYLAMIDE, ET UTILISATION CORRESPONDANTE

(30) Priority: 27.11.2002 JP 2002344613; 24.12.2002 JP 2002372115; 26.12.2002 JP 2002378582; 04.02.2003 JP 2003027433; 04.03.2003 JP 2003057582; 26.03.2003 JP 2003086324; 29.07.2003 JP 2003282087
(43) Date of publication of application: 31.08.2005
(73) Proprietor: KABUSHIKI KAISHA HAYASHIBARA SEIBUTSU KAGAKU KENKYUJO, Okayama-shi, Okayama 700-0907 (JP)
(72) Inventor: OKU, Kazuyuki, c/o K. K. Hayashibara Seibutsu, Okayama-shi, Okayama 700-0907 (JP); KUBOTA, Michio, c/o K. K. Hayashibara Seibutsu, Okayama-shi, Okayama 700-0907 (JP); FUKUDA, Shigeharu, c/o K. K. Hayashibara Seibutsu, Okayama-shi, Okayama 700-0907 (JP); MIYAKE, Toshio, c/o K. K. Hayashibara Seibutsu, Okayama-shi, Okayama 700-0907 (JP)
(74) Representative: Daniels, Jeffrey Nicholas
(86) International application number: PCT/JP2003/014819
(87) International publication number: WO 2004/047559

(56) References cited:
- WO-A-20/04032647
- JP-A- 11 290 031
- JP-A- 2000 004 836
- JP-A- 2000 175 669
- JP-A- 2000 300 190

## Description

### TECHNICAL FIELD

The present invention relates to a method for suppressing the formation of acrylamide which is known as a carcinogen or a substance causing nerve disorder, and its uses. Particularly, the present invention relates to a method for suppressing the formation of acrylamide formed by heating edible materials, comprising organic substances having an amino group(s) and/or saccharides having an ability of forming acrylamide, at a relatively high temperature; more particularly, a method for suppressing the formation of acrylamide by admixing an organic substance(s), having an ability of suppressing the formation of acrylamide, with an edible material, having a possibility of forming acrylamide by heating, before heating; and its uses.

### BACKGROUND ART

Recently, Eden Tareke et al. reported in Journal of Agricultural and Food Chemistry, Vol. 50, pp. 4998-5006 (2002) that relatively large amount of acrylamide which is known as a carcinogen is contained in processed foods, which are frequently ingested in our daily life, such as potato chips, fried potatoes, breads, fried buns, etc. Therefore, the influence of acrylamide on our health has been apprehensive.

A mechanism of the formation of acrylamide was suggested by Donald S. Mottram et al. in Nature, Vol. 419, pp.448-450 (2002). They suggested that acrylamide was formed from amino acids such as L-asparagine and reducing saccharides such as D-glucose, both comprised in food materials, by Maillard reaction caused by heating at a relatively high temperature over 100 DEG C.

As described above, it was found that acryl amide was formed in the process of heating edible materials. However, a method for reducing the amount of acrylamide or suppressing the formation of acrylamide has not been developed. Therefore, the establishment of such method is desired. Such a circumstance can be understood by the manifestation of "Researches for suppressing the formation of acrylamide and the toxicity is executed quickly" in website of Ministry of Health, Labour, Welfare, "Acrylamide in Processed Foods", published by Department of Food Health, topics on October, 31, 2002, http://www.mhlw.go.jp./houdou/2002/10/h1031-2.html.

WO 2004/032647 discloses the use of flavonoids such as kaempherols, quercetins, myricetins and flavonols such as epigallocatechins, epigall ocatechin gallates, (-)-catechins and epicatechin gallates and their derivates in the prevention of acrylamide formation in foods.

JP 11-290031 discloses tartaric acid treatments for reducing the fishy smell of foods.

JP 2000-175669 discloses that meat, seafood and vegetables can be heat treated by immersion into a liquid containing 1 to 40% trehalose.

### DISCLOSURE OF INVENTION

The object of the present invention is to establish a method for suppressing the formation of acrylamide formed by heating edible materials at a relatively high temperature and to provide compositions, having a satisfactory safety, such as foods, beverages, cosmetics, pharmaceuticals, and intermediates thereof.

Accordingly, the present invention provides a method for suppressing the formation of acrylamide, comprising the steps of incorporating a saccharide and/or phenol, having an ability of suppressing the formation of acrylamide, into an edible material having a possibility of forming acrylamide by heating; and heating the resulting mixture; said saccharide having an ability of suppressing the formation of acrylamide is one or more saccharides selected from the group consisting of α,α-trehalose, α,β-trehalose, reduced palatinose, α-glucosyl α,α-trehalose, α-maltosyl α,α-trehalose, D-mannitol, D-erythritol, and β-cyclodextrin; and said phenol having an ability of suppressing the formation of acrylamide is one or more phenols selected from the group consisting of cinnamic acid, quinic acid, 3,4-dihydro-cinnamic acid, 3-coumaric acid, 4-coumaric acid, *p*-nitrophenol, curcumin, scopoletin, *p*-hydroxybenzoic acid n-propyl, and saccharide derivatives thereof.

To solve the above object, the present inventors have been extensively studied on the effects of various saccharides on the formation of acrylamide from an organic substance (s) under the condition at high temperatures. As a result, the present inventors found a novel phenomenon that the formation of acrylamide from an organic substance (s) having an amino group (s) and saccharide (s) having an ability of forming acrylamide by reacting with the organic substance (s) having an amino group (s) by heating can be remarkably suppressed by incorporating specific organic substances before heating in comparison with the case without such specific substances The specific organic substances described above are, particularly, saccharides and/or phenolic substances, having an ability of suppressing the formation of acrylamide; more particularly, one or more saccharides selected from the group consisting of α,α-trehalose, α,β-trehalose, reduced palatinose, α-glucosyl α,α-trehalose, α-maltosyl, α,α-trehalose, D-mannitol, D-erythritol, and β-cyclodextrin; and/or one or more phenolic substances selected from the group consisting of cinnamic acid, quinic acid, 3,4-dihydro-cinnamic acid, 3-coumaric acid, 4-coumaric acid, *p*-nitrophenol, curcumin, scopoletin, p-hydroxybenzoic acid n-propyl, and those saccharide derivatives. The present inventors accomplished the present invention on the basis of above knowledge.

The present invention solves the above object by establishing a method for suppressing the formation of acrylamide which comprises the steps of incorporating an organic substance (s), having an ability of suppressing the formation of acrylamide, into an edible material, having a possibility of forming acrylamide by heating, before heating and heating; more particularly, a method for suppressing the formation of acrylamide which comprises the steps of incorporating an organic substance(s), having an ability of suppressing the formation of acrylamide, into an edible material, having a possibility of forming acrylamide by heating, which contains an organic substance(s) having an amino group(s) and a saccharide(s) having an ability of forming acrylamide by reacting with the organic substance (s) having an amino group(s), before heating and then heating. The present invention also solves the above object by providing compositions whose possibility of forming acrylamide is suppressed such as foods, beverages, cosmetics, pharmaceuticals, and intermediates thereof, produced by the above method, agents for suppressing the formation of acrylamide which contain an organic substance(s) having an ability of suppressing the formation of acrylamide as an effective ingredient (s) , and compositions having an ability of suppressing the formation of acrylamide produced by incorporating an organic substance(s) having an ability of suppressing the formation of acrylamide, such as foods, beverages, cosmetics, pharmaceuticals, and intermediates thereof.

According to the method for suppressing the formation of acrylamide of the present invention, the formation of acrylamide from an organic substance (s) having an amino group (s) which is contained in or incorporated into an edible material (s) and a saccharide (s) having an ability of forming acrylamide by reacting with the organic substance (s) having an amino group (s) can be remarkably suppressed in the case of heating an edible material (s) having a possibility of forming acrylamide by heating.

### BEST MODE FOR CARRYING OUT THE INVENTION

An edible material having a possibility of forming acrylamide by heating as referred to as in the present invention means every edible material containing and/or being incorporated with an organic substance(s) having an amino group(s) and/or a saccharide(s) having an ability of forming acrylamide by reacting with the organic substance (s) having an amino group (s), and having a possibility of forming acrylamide by heating.

The organic substance having an amino group(s) as referred to as in the present invention includes, for example, amino acid selected from the group consisting of glycine, L-alanine, L-valine, L-leucine, L-isoleucine, L-serine, L-threonine, L-aspartic acid, L-glutamic acid, L-asparagine, L-glutamine, L-lysine, L-arginine, L-cysteine, L-methionine, L-phenylalanine, L-tryptophan, and L-proline, and those salts, and peptides and proteins constructed by those amino acids.

The edible material containing an organic substance(s) having an amino group (s) includes agricultural-, stockbreeding- or marine- products or processed products of those containing one or more organic substances having an amino group(s) as described above. More concretely, the edible material containing an organic substance (s) having an amino group (s) includes amino acid- or nucleic acid-seasonings comprising an organic substance (s) having an amino group(s) as described above, and seasonings such as soy sauce, *"miso* (soybean paste)", sauce, sauce for *"yakiniku* (roasted meat)", ketchup, mayonnaise, butter, cheese, vinegar, *"mirin* (sweetened sake)", sake, wine, curry roux, stew premix, bouillon premix, oyster extract, Skipjack extract, "*kombu* (kelp) "extract, *"shiitake"* mushroom extract, yeast extract, and meat extract.

The saccharide having an ability of forming acrylamide as referred to as in the present invention means every saccharide having a possibility of forming acrylamide by reacting with an organic substance having an amino group (s). Such a saccharide includes one or more monosaccharides selected from the group consisting of D-xylose, L-arabinose, D-glucose, D-fructose, and D-galactose; reducing oligosaccharides containing one or more saccharides selected from the group consisting of D-xylose, L-arabinose, D-glucose, D-fructose, and D-galactose as a component sugar; non-reducing oligosaccharides, containing D-frucrose as a component sugar, which is easily decomposed by acid or heating, such as sucrose, raffinose, stachyose; and the genus L-ascorbic acid such as L-ascorbic acid, L-ascorbic acid derivatives, and those salts. Salts of L-ascorbic acid or its derivatives include alkali metal salts such as sodium salt and alkali earth metal salts such as magnesium salt.

The edible material containing an saccharide (s) having an ability of forming acrylamide includes agricultural, stockbreeding or marine products or processed products of those containing one or more saccharides described above. More concretely, an edible material containing a saccharide (s) having an ability of forming acrylamide includes saccharides described above, and sweeteners such as D-glucose, D-fructose, isomerized sugar, starchy syrup, reduced starchy syrup, lactose and sucrose.

In addition to the seasonings and sweeteners described above, concrete examples of foods and beverages as an edible material comprising an organic substance(s) having an amino group(s) and/or a saccharide(s) having an ability of forming acrylamide by reacting with the organic substance (s) having an amino group (s), include foods such as sliced potatoes, boiled potatoes, steamed potatoes, mashed potatoes, shaped reconstituted potatoes, potato powder, germinated wheat, crushed wheat, flour, wheat germ, dough, gluten, germinated barley, germinated rice, rice grits, rice flour, corn grits, corn flour, buckwheat flour, soybean flour, defatted soybean, soybean casein, boiled soybean, soybean paste, germinated soybean; agricultural products and those processed products, comprising premix and intermediate products produced by incorporating one or more above materials, such as sponge mix, doughnut mix, flour for *"tempura"*, set of ingredients for *"tempura"*, and salad mix; stockbreeding products and those processed products such as chilled or frozen meat, dressed carcuss, milk, skim milk, fresh cream, milk casein, butter, cheese, whey, whole egg, liquid egg, egg yolk, egg white, and boiled egg; and marine products and those processed product such as chilled or frozen fish, fillet, minced fish, dried marine products, salt cured marine product, and flesh of shellfish.

The organic substance having an ability of suppressing the formation of acrylamide as referred to as in the present invention means every organic substance showing an activity of suppressing the formation of acrylamide by incorporating into edible material containing aforesaid organic substance(s) having an amino group(s) and aforesaid saccharide(s) having an ability of forming acrylamide before heating. The organic substance having an ability of suppressing the formation of acrylamide includes saccharides and/or phenolic substances, both having an ability of suppressing the formation of acrylamide. For example, saccharides such as α,α-trehalose, α,β-trehalose, reduced palatinose, α-glucosyl α,α-trehalose, α-maltosyl α,α-trehalose, D-mannitol, D-erythritol, β-cyclodexrin, etc. can be used as a saccharide having an ability of suppressing the formation of acrylamide. Also, for example, phenolic substances such as cinnamic acid, quinic acid, 3,4-dihydro-cinnamic acid, 3-coumaric acid, 4-coumaric acid, *p*-nitrophenol, curcumin, scopoletin, p-hydroxybenzoic acid n-propyl, protoanthocyanidin; and those saccharide derivatives can be used as a phenolic substance having an ability of suppressing the formation of acrylamide. One or more organic substances selected from the above can be advantageously used in the present invention. Especially, the formation of acrylamide, by heating edible material comprising an organic substance(s) and a saccharide(s) having an ability of forming acrylamide, can be remarkably suppressed by saccharides such as α,α-trehalose and/or α,β-trehalose and phenolic substances such as rutin, naringin, and/or hesperidin.

Also, commercially available saccharides can be advantageously used as the above saccharides having an ability of suppressing the formation of acrylamide. For example, "TREHA®", a high purity hydrous crystalline α,α-trehalose commercialized by Hayashibara Shoji Inc., Okayama, Japan, "NEOTREHALOSE", a reagent grade crystalline α,β-trehalose commercialized by Hayashibara Biochemical Laboratories Inc., Okayama, Japan, "PALATINIT", a powderized reduced palatinose commercialized by Shin Mitsui Sugar Co. Ltd., Tokyo, Japan, "ISOMALDEX", a powderized reduced palatinose commercialized by Cerestar Japan, Tokyo, Japan, "MANNITOL", a crystalline mannitol powder commercialized by Towa Chemical Industry Co., Ltd., Tokyo, Japan, "ERYTHRITOL", a crystalline erythritol commercialized by Mitsubishi-Kagaku Foods Corporation, Tokyo, Japan, "ERYSWEET", a crystalline erythritol powder commercialized by Nikken Chemical Co., Ltd., Tokyo, Japan, and "DEXYPEARL β-100", a high purity β-cyclodextrin commercialized by Bio Research Corporation of Yokohama, Kanagawa, Japan can be advantageously used.

In addition to commercially available reagent grade phenolic substances such as cinnamic acid, quinic acid, 3,4-dihydro-cinnamic acid, 3-coumaric acid, 4-coumaric acid, *p*-nitorophenol, curcumin, scopoletin, *p*-hydroxybenzoic acid n-propyl, protoanthocyanidin; and those saccharide derivatives, and mixtures and saccharide derivatives therof, commercialized as healthy foods, can be advantageously used as phenolic substances having an ability of suppressing the formation of acrylamide in the present invention.

In the case of using an saccharide(s) as an organic substance(s) having an ability of suppressing the formation of acrylamide, it is desirable in the present invention to incorporate the saccharide (s) in a molar ratio of, usually, 0.5 or higher, preferably, 0.75 or higher, more preferably, 1.0 or higher to that of an organic substance(s) having an amino group (s) or a saccharide (s) having an ability of forming acrylamide in order to express the activity of suppressing the formation of acrylamide. In the case of using the saccharide (s) in the molar ratio of less than 0.5, it is possible that the activity of suppressing the formation of acrylamide is not expressed fully. While, in the case of using a phenolic substance(s) as an organic substance (s) having an ability of suppressing the formation of acrylamide, it is desirable to incorporate the phenolic substance(s) in a molar ratio of, usually, 0.004 or higher, preferably, 0.01 or higher, more preferably, 0.02 or higher to that of an organic substance (s) having an amino group (s) or a saccharide(s) having an ability of forming acrylamide. In the case of using the phenolic substance(s) in the molar ratio of less than 0.004, it is possible that the activity of suppressing the formation of acrylamide is not expressed fully.

The heating treatment as referred to as in the present invention means that at a temperature (s) of forming acrylamide by reacting an organic substance (s) having an amino group (s) and a saccharide (s) having an ability of forming acrylamide, both comprised in an edible material(s), wherein the temperature is, usually, 80°C or higher, specifically, about 100 to 240°C. Method for heating the material is not specifically restricted. For example, such a method includes baking, frying, roasting, heating with microwave oven, heating in retort pouch, heating by pressurizing, boiling, steaming, simmering, heating for sterilization, etc.

Usually, the amount of acrylamide formed from an organic substance (s) having an amino group (s) and a saccharide (s) having an ability of forming acrylamide, both comprised in edible materials, increases with the increase of heating temperature or of heating time. In addition, as shown in the following experiments, the amount of acrylamide increases as the decrease of moisture content of materials (edible materials) even in the case of the same heating temperature and heating time. The organic substance (s) having an ability of suppressing the formation of acrylamide of the present invention can suppress the formation of acrylamide even in the case of the moisture content of samples (edible materials) being high or low. Specifically, in order to suppress the formation of acrylamide remarkably, the moisture content of samples (edible materials) to be heated is preferable to control, usually, to a level of about 10% (w/w) or higher.

Various compositions, such as foods, beverages, cosmetics, pharmaceuticals, and intermediates thereof, whose possibility of forming acrylamide is suppressed, canbe produced by using the method for suppressing the formation of acrylamide of the present invention. To produce various compositions such as foods, beverages, cosmetics, and pharmaceuticals, an organic substance(s) having an ability of suppressing the formation of acrylamide can be incorporated into an edible material (s) before heating, i.e., in the process of mixing materials for preparing edible material or just before heating. A method for incorporating the organic substance (s) can be arbitrary selected from, for example, mixing, kneading, dissolving, dispersing, suspending, emulsifying, penetrating, applying, spraying, coating, injecting, soaking, etc. Optionally, plant cells, such as fruits and vegetables, which are allowed to comprise an organic substance (s) having an ability of suppressing the formation of acrylamide, for example, saccharides or phenolic substances, by genetic manipulation, can be advantageously used as such edible materials.

Agents for suppressing the formation of acrylamide can be advantageously produced according to the present invention by incorporating an organic substance(s) having an ability of suppressing the formation of acrylamide, i.e., one ore more saccharides selected from the group consisting of α,α-trehalose, α,β-trehalose, reduced palatinose, α-glucosyl α,α-trehalose, α-maltosyl α,α-trehalose, D-mannitol, D-erythritol, and β-cyclodextrin; and/or one ore more phenolic substances selected from the group consisting of cinnamic acid, quinic acid, 3,4-dihydro-cinnamic acid, 3-coumaric acid, 4-coumaric acid, *p*-nitrophenol, curcumin, scopoletin, p-hydroxybenzoic acid n-propyl, protoanthocyanidin; and those saccharide derivatives; into other materials.

According to the present invention, a composition comprising edible materials, having an ability of suppressing the formation of acrylamide can be advantageously produced by incorporating the aforesaid organic substance (s) having an ability of suppressing the formation of acrylamide into an edible material (s) having an possibility of forming acrylamide by heating.

Also, a composition having an ability of suppressing the formation of acrylamide can be advantageously produced by using the aforesaid saccharide (s) and/or phenolic substance (s), having an ability of suppressing the formation of acrylamide and the aforesaid organic substance (s) having an amino group (s) and/or the aforesaid succharide (s) having an ability of forming acrylamide in combination. More particularly, in the case of the organic substance(s) having an amino group(s) being one ore more organic substances selected from the group consisting of amino acids such as glycine, L-alanine, L-valine, L-leucine, L-isoleucine, L-serine, L-threonine, L-aspartic acid, L-glutamic acid, L-asparagine, L-glutamine, L-lysine, L-arginine, L-cysteine, L-methionine, L-phenylalanine, L-tryptophan, L-proline, and those salts; peptides or proteins, constructed by those amino acids; and in the case of the saccharide having an ability of forming acrylamide being one or more saccharides selected from the group consisting of monosaccharide such as D-xylose, L-arabinose, D-glucose, D-fructose, and D-galactose; reducing oligosaccharides comprising one ore more saccharides selected from the group consisting of D-xylose, L-arabinose, D-glucose, D-fructose, and D-galactose as component sugars; non-reducing oligosaccharides comprising D-fructose as a component sugar; and the genus L-ascorbic acid; the amount of saccharide (s) and/or phenolic substance (s), having an ability of suppressing the formation of acrylamide, required for suppressing the formation of acrylamide is varied depending on the amount of such organic substances having an amino group(s) and that of such saccharide(s) having an ability of forming acrylamide. However, a composition having an ability of suppressing the formation of acrylamide such as seasonings and saccharides can be produced by incorporating α,α-trehalose and/or α,β-trehalose in a molar ratio of, usually, 0.5 or higher, preferably, 0.75 or higher, more preferably, 1.0 or higher as saccharide having an ability of suppressing the formation of acrylamide, and/or rutin, naringin, and/or hesperidin in a molar ratio of, usually, 0.004 or higher, preferably, 0.01 or higher, more preferably, 0.02 or higher as a phenolic substance having an ability of suppressing the formation of acrylamide, into such substances having an amino group(s) or an ability of forming acrylamide. Such a composition has a satisfactory effect of remarkably suppressing or substantially stopping the formation of acrylamide when it is heated intact or together with other materials.

A composition comprising L-ascorbic acid or its derivatives for various uses, having an ability of suppressing the formation of acrylamide, preferably, a solid composition such as a powder, granule, tablet, and the like, having a satisfactory handleability and stability for preservation, can be advantageously produced by incorporating the aforesaid organic substance(s) having an ability of suppressing the formation of acrylamide into L-ascorbic acid or its derivatives. Such a composition comprising L-ascorbic acid or its derivatives can effectively suppress the formation of acrylamide by using for the production of compositions, requiring L-ascorbic acid or its derivatives and comprising edible materials having a possibility of forming acrylamide by heating. Such a composition comprising L-ascorbic acid or its derivatives can be advantageously used for producing various high-quality compositions such as foods, beverages, cosmetics, pharmaceuticals, and intermediates thereof, which are supplemented with L-ascorbic acid (vitamin C) or its derivatives and have a satisfactory safety.

Such foods include various foods produced by heating for animals including humans and pets, for example, fried confectionery such as potato chips, fried potatoes, fried onion, corn chips, banana chips, vegetable chips, fried *"senbei"* (rice cake), *"daigaku imo"* (fried sweet potatoes coated with syrup), *"imo-kenpi"* (fried and sweetened sweet potatoes), *"karintou"* (fried and sweetened cake), snack, and the like; fried food such as croquette, cutlet, fry, *"tempura", "abura-age"* (friedbeancurd), *"satsuma-age"* (fried minced fish), *"agedama", "atsu-age"* (fried bean curd), *"harumaki"* (spring roll), fried noodles (instant noodles), and the like; Japanese confectionery such as *"senbei"* (rice cake), *"arare"* (rice cake), *"dorayaki"* (sweetened bean jam cake), *"monaka"* (sweetened bean jam cake), *"manju"* (sweetened bean jam cake), *"taiyaki"* (sweetened bean jam cake), *"takoyaki"* (sliced octopus cake), *"mugikogashi"* (roasted barley powder), and the like; confectionery or snack such as biscuit, cracker, cookie, pretzel, cereals, popcorn, and the like; processed food of wheat flour such as bread, pizza, pie, sponge cake, waffle, pao de Castella, doughnut, and the like; cooked meat such as ham, sausage, and the like, cooked egg such as scrambled egg, *"dashi-maki-tamago"* (baked egg with soup stock), *"kinshi-tamago"* (sliced baked egg), boiled egg, and the like; cooked marine product such as steamed fish paste, *"chikuwa"* (fish paste cooked in a bamboo-like shape), *"hanpen"* (a steamed fished paste), fish-sausage, and the like; dish such as grilled meat, grilled chicken, grilled pork, grilled fish, grilledrice ball, grilled rice cake, Chinese fried rice, hamburger steak, dumpling, gratin, hashed rice, curry rice, risotto, quiche, and the like, beverage such as beverage containing amino acids, beverage containing peptides, beverage containing soymilk, beverage containing milk, beverage containing lactic acid, beverage containing fruits juice, beverage containing vegetables juice, cocoa, coffee, *"amazake"* (a sweet beverage made from fermented rice or sake lees), *"shiruko"* (rice cake with bean jam), and the like; tea such as barley tea, *"houji-tya"* (roasted tea), Chinese tea, black tea, and the like; juice made from fruits, vegetables, and herbs; processed fruits or vegetables such as dry fruits, preserves, sauce, chutney, jam, marmalade, spread, jelly, candy, canned food, bottled food, boiled food, fry, baked food, and the like; roasted processed food such as sweet roasted chestnuts, peanut, almond, chocolate powder, coffee powder, coffee, cocoa, curry powder, roasted sesame, roasted rice, roasted barley, sweet roasted barley powder, roasted soybean powder, roasted wheat germ, roasted rice germ, and the like; seasoned food of agricultural products, processed meat, processed egg, and processed marine product and those canned or bottled; pet food, feed, or bait such as cookie, biscuit, beef jerky, processed vegetables, and the like. In those foods and beverages, obtainable by using the method for suppressing the formation of acrylamide of the present invention, the amount of acrylamide formed by heating is remarkably suppressed. Therefore, they can be advantageously used as foods and beverages having a satisfactory safety and low fear of causing cancer and nerve disease, usable in daily life.

A cosmetic product, which can be produced by applying the method for suppressing the formation of acrylamide of the present invention, having the form of a lotion, cream, emulsion, gel, powder, paste, block, or the like, includes clean up cosmetics such as a soap, cosmetic soap, powdery body-wash, cleansing cream, cleansing foam, facial rinse, body shampoo, body rinse, shampoo, rinse, powdery hair-wash; hair cosmetic such as set lotion, hair blow, stick, hair cream, pomade, hair spray, hair liquid, hair tonic, hair lotion, hair dye, scalp treatment, and the like; basic cosmetic such as lotion, vanishing cream, emollient cream, emollient lotion, pack (peel-of type jelly, wipe-out type jelly, wash-out type paste, powder, and the like), cleansing cream, cold cream, hand cream, hand lotion, emulsion, moisture-retaining lotion, after-shaving lotion, shaving lotion, pre-shave lotion, after-shaving cream, after-shaving foam, pre-shave cream, cosmetic oil, baby oil, and the like; cosmetic for make-up such as foundation (in a form of liquid, cream, or solid), talcum powder, baby powder, body powder, perfume powder, make-up base, face powder (in a form of cream, paste, liquid, solid, powder, and the like), eye shadow, eye cream, mascara, eyebrow pencil, cosmetic for eyelash, cheek rouge, cosmetic lotion for cheek, and the like; fragrance such as perfume, paste perfume, powdery perfume, eau de cologne, perfume cologne, eau de toilette, and the like; cosmetic for sunburn or sunscreen such as sunburn cream, sunburn lotion, sunburn oil, sunscreen cream, sunscreen lotion, sunscreen oil, and the like; cosmetic for nail care such as manicure, pedicure, nail color, nail lacquer, enamel remover, nail cream, nail lotion, and the like; eye liner; cosmetic for lip such as lipstick, lip cream, rouge, lip gross, and the like; oral cosmetic such as tooth paste, mouth wash, and the like; bath cosmetic such as bath salt, bath oil, bath lotion, and the like. In those cosmetics, obtainable by using the method for suppressing the formation of acrylamide of the present invention, the amount of acrylamide formed by heating is remarkably suppressed. Therefore, they can be advantageously used as cosmetics having a satisfactory safety and low fear of causing cancer and nerve disease, usable in daily life.

A pharmaceutical product, which can be produced by applying the method for suppressing the formation of acrylamide of the present invention, includes drinkable preparations, extracts, elixirs, capsules, granules, pills, ophthalmic ointments, oral mucosa plasters, suspensions, emulsions, plasters, suppositories, powders, spirits, tablets, syrups, injections, tinctures, ophthalmic solutions, eardrops, collunariums, troches, ointments, aromatic waters, nose aerosols, limonades, liniments, fluidextracts, lotions, aerosols, bath agents, plasters, cataplasms, etc. In those pharmaceuticals, obtainable by using the method for suppressing the formation of acrylamide of the present invention, the amount of acrylamide formed by heating is remarkably suppressed. Therefore, they can be advantageously used as pharmaceuticals having a satisfactory safety and lesser fear of causing cancer and nerve disease, applicable in daily life.

The following experiments explain the present invention in detail:

### Experiment 1

### Formation of acrylamide from an amino acid and various saccharides by heating

The formation of acrylamide from an amino acid and various saccharides by heating was examined using L-asparagine as the amino acid, according to the method of Donald S. Mottram et al. disclosed in Nature, Vol. 419, pp. 448-450 (2002) as follows : Onemmol eachofD-glucose, D-fructose, D-galactose, α,α-trehalose, α,β-trehalose, maltose, sucrose, lactose, and L-ascorbic acid was weighed and placed in a 20 ml-tube with a stopper made by ground glass containing one mmol of L-asparagine, respectively, and then one ml of 0.5 M sodium phosphate buffer (pH6.0) to respective tubes. Successively, each tube was sealed with the stopper and heated in an oil bath at 150°C for 20 minutes. After heating, each tube was immediately cooled to ambient temperature in running water. The amount of acrylamide formed in each sample was determined by the steps of converting acrylamide into 2-bromopropenamide-derivative according to the method described in "Guideline of examination method for evaluating chemicals for water" (Water Supply Division, Water Supply and Environment Sanitation Department, Life Bureau, Ministry of Health and Welfare, March, 2000) and determining a peak, showing an identical retention time with the standard acrylamide derivative, as the derivative derived from acrylamide in each sample by using gas-chromatography.

Gas chromatographic analysis was carried out under the following conditions:

### (Conditions of gas chromatography)

Gas chromatograph: GC-14A commercialized by Shimadzu Corporation, Kyoto, Japan
Column: TC-FFAP capillary column, 0.53 mm i.d. x 30 m,
   film thickness 1 µm,
   commercialized by GL Sciences, Tokyo, Japan
Column temperature: After keeping 120°C for 5 minutes, column was heated to 240°C with a rising speed of 7.5°C/min
Carrier gas: helium (one ml/min)
Detection: FID (Flame-ionization detector)
Temperature of sample injection port: 250°C
Sample injection volume: 4 µl (whole)

The results are in Table 1. The relative amount of acrylamide (%) was shown by relative value calculated from the amount of acrylamide formed from D-glucose as 100%. Throughout Experiments 1 to 10, the amount of formed acrylamide (mg) in table is expressed as the amount of acrylamide formed in the presence of one mole L-asparagine.

**Table 1**

| Saccharide | Acrylamide | |
|---|---|---|
| | Formed amount (mg) | Relative amount (%) |
| D-Glucose | 54.3 | 100 |
| D-Fructose | 71.9 | 132 |
| D-Galactose | 22.1 | 41 |
| α,α-Trehalose | 0.0 | 0 |
| α,β-Trehalose | 0.0 | 0 |
| Maltose | 10.8 | 20 |
| Sucrose | 10.6 | 19 |
| Lactose | 15.3 | 28 |
| L-Ascorbic acid | 235.0 | 433 |

As shown in Table 1, a relatively large amount of acrylamide was formed from D-glucose, D-fructose, and L-ascorbic acid by heating in the presence of L-asparagine. Specifically, the amount of acrylamide formed from L-ascorbic acid was four-folds or more than that from D-glucose. Although the amount of acrylamide was lower in comparison with the cases of D-glucose and D-fructose, acrylamide was formed from D-galactose, maltose, sucrose, and lactose by heating with L-asparagine. On the contrary, no acrylamide was formed fromα,α-trehalose and L-asparagine, and α,β-trehalose and L-asparagine by heating. It has been known that α,α-trehalose and α,β-trehalose, saccharides belonging to non-reducing saccharides, hardly react with amino acids by Maillard reaction. Such reactivity was also confirmed by the results shown in Table 1. However, in the case of sucrose which also belongs to non-reducing saccharides, acrylamide was formed. The reason of the formation of acrylamide from sucrose was supposed that sucrose was easily decomposed by heating, and the resulting D-glucose and D-fructose, formed by decomposing sucrose by heating, reacted with L-asparagine. The following Experiments 2-8 and 9-12 describe experiments using D-glucose and L-ascorbic acid as a saccharide having an ability of forming acrylamide by heating with L-asparagine, respectively.

### Experiment 2

### Effects of various saccharides on the formation of acrylamide from L-asparagine and D-glucose

Effects of various saccharides on the formation of acrylamide from L-asparagine and D-glucose by heating were examined. One mmol of any saccharide shown in Table 2 was placed in a 20 ml-tube with ground glass stopper, containing one mmol of L-asparagine and one mmol of D-glucose. In the cases of α-cyclodextrin and β-cyclodextrin, 720 mg each (corresponding to about 0.63 or 0.74 mmol) of saccharide was used. Except for the condition described above, samples were treated with the procedure in Experiment 1 and the amounts of formed acrylamide were determined. A sample, containing L-asparagine and D-glucose, was examined as a control without admixing with any other saccharide. The results are in Table 2. Each relative amount of acrylamide (%) is expressed in a relative value calculated using the amount of acrylamide in a control sample as 100%.

**Table 2**

| Saccharide | Acrylamide | |
|---|---|---|
| | Formed amount (mg) | Relative amount (%) |
| Control (L-Asn* + D-Glucose) | 52.3 | 100 |
| D-Fructose | 64.4 | 123 |
| D-Sorbitol | 49.4 | 95 |
| D-Mannitol | 19.6 | 38 |
| D-Erythritol | 20.5 | 39 |
| D-Xylitol | 41.9 | 80 |
| D-Galactose | 63.1 | 121 |
| L-Arabinose | 48.0 | 92 |
| α,α-Trehalose | 7.2 | 14 |
| α,β-Trehalose | 8.1 | 15 |
| Kojibiose | 31.2 | 60 |
| Nigerose | 29.3 | 56 |
| Maltose | 46.6 | 89 |
| Isomaltose | 31.5 | 60 |
| Maltitol | 40.9 | 78 |
| Isomaltitol | 59.1 | 113 |
| Sucrose | 29.4 | 56 |
| Palatinose | 28.5 | 54 |
| Reduced Palatinose | 22.6 | 43 |
| Lactose | 33.3 | 64 |
| Lactitol | 41.3 | 79 |
| Maltotriose | 47.9 | 92 |
| α-Glucosyl α,α-trehalose | 24.4 | 47 |
| Lactosucrose | 34.8 | 67 |
| Maltotetraose | 37.1 | 71 |
| α-Maltosyl α,α-trehalose | 31.3 | 60 |
| Cyclic tetrasaccharide** | 42.1 | 81 |
| α-Cyclodextrin | 62.1 | 119 |
| β-Cyclodextrin | 20.2 | 39 |

| | | |
|---|---|---|
| *: L-Asparagine **:Cyclic tetrasaccharide constructed by 4 glucose molecules bound together by alternating α-1,6 and α-1,3 glucosidic linkages. | | |

As is evident from the results in Table 2, it was revealed that the formation of acrylamide from L-asparagine and D-glucose by heating was remarkably suppressed in the presence of some saccharides. Particularly, the formation of acrylamide from L-asparagine and D-glucose was suppressed to the level of less than 50% by α,α-trehalose, α,β-trehalose, reduced palatinose, α-glucosyl α,α-trehalose, D-mannitol, D-erythritol, and β-cyclodextrin. More particularly, it was confirmed that the relative amount of acrylamide was significantly suppressed to the level of less than 20%. The above all saccharides, having an ability of suppressing the formation of acrylamide, belong to non-reducing saccharides. It has been known that non-reducing saccharides hardly causes Maillard-reaction with organic substances having an amino group (s). However, it is a information beyond our expectation that non-reducing saccharides remarkably suppress the formation of acrylamide on the reaction of a reducing saccharide and an organic substance having an amino group(s) and show remarkable effectiveness.

### Experiment 3

### Effects of the amount of D-glucose and the presence of α,α-trehalose on the formation of acrylamide from L-asparagine and D-glucose

Effects of the amount of D-glucose and the presence of α,α-trehalose, which remarkably suppressed the formation of acrylamide in Experiment 2, on the formation of acrylamide from L-asparagine and D-glucose were examined. Except for adding 0.01, 0.10, 0.25, 0.50, 1.00, 1.33, or2.00mmolofD-glucose to a tube with a ground glass stopper containing one mmol of L-asparagine, samples were treated with the same method in Experiment 1 and the amount of formed acrylamide was determined, respectively. In addition, samples prepared by adding one mmol of α,α-trehalose to the above samples were also tested. The results are in Table 3. In the table, the molar ratio means that of α,α-trehalose to D-glucose, and the percentage of acrylamide formation (%) means each relative amount of acrylamide formed in the presence of α,α-trehalose calculated by using the amount of acrylamide (mg) formed in the absence of α,α-trehalose as 100% for each sample.

**Table 3**

| D-Glucose (mmol) | Formed acrylamide (mg) | | Molar ratio (Tre/Glc) | Percentage of Acrylamide Formation (%) |
|---|---|---|---|---|
| | Tre (-) * | Tre (+) ** | | |
| 0.01 | 5.2 | 1.0 | 100 | 18 |
| 0.10 | 20.2 | 3.9 | 10 | 19 |
| 0.25 | 35.2 | 4.9 | 4 | 14 |
| 0.50 | 52.0 | 5.6 | 2 | 11 |
| 1.00 | 52.3 | 7.2 | 1 | 14 |
| 1.33 | 52.5 | 11.6 | 0.75 | 22 |
| 2.00 | 53.0 | 25.2 | 0.5 | 48 |

| | | | | |
|---|---|---|---|---|
| Tre: α,α-trehalose Glc: D-glucose *: in the absence of α,α-trehalose **: in the presence of α,α-trehalose | | | | |

As is evident from the results in Table 3, in the absence of α,α-trehalose, the amount of acrylamide formed from L-asparagine increased as the increase of the amount of D-glucose. The amount of acrylamide formed from one mmol of L-asparagine showed almost constant value in the range of the amount of D-glucose of 0.50 to 2.00 mmol. While, in the presence of α,α-trehalose, although the amount of acrylamide increased as the increase of the amount of D-glucose, but the level of acrylamide was low. In the case of the molar ratio of α,α-trehalose to D-glucose was 0.5 or higher, percentage of acrylamide formation (%) in the presence of α,α-trehalose was less than 50% compared with that in the absence of α,α-trehalose. Particularly, in the case of the molar ratio of α,α-trehalose to D-glucose was 0.75 or higher, the percentage of acrylamide formation was less than 25%. More particularly, in the case of the molar ratio was one or higher, the percentage of acrylamide formation was less than 20%. Therefore, it was confirmed that α,α-trehalose suppressed the formation of acrylamide remarkably.

### Experiment 4

### Effects of the amount of α,α-trehalose on the formation of acrylamide from L-asparagine and D-glucose

The dose dependency of the ability of suppressing the formation of acrylamide was examined using α,α-trehalose which showed remarkable activity of suppressing the formation of acrylamide from L-asparagine and D-glucose by heating in Experiment 2. Except for adding 0.00, 0.10, 0.25, 0.50, 0.75, 1.00, 1.50, or 2.00 mmol of α,α-trehalose to a 20 ml-tube with a ground glass stopper containing one mmol each of L-asparagine and D-glucose, samples were treated with the same method as in Experiment 1 and determined for the amount of formed acrylamide, respectively. The results are in Table 4. In the table, each relative amount of acrylamide (%) was calculated by using the amount of acrylamide (mg) formed in the absence of α,α-trehalose as 100% for each sample.

**Table 4**

| Amount of α,α-trehalose (mmol) | Molar ratio (Tre/Glc) | Acrylamide | |
|---|---|---|---|
| | | Formed amount (mg) | Relative amount (%) |
| 0.00 | 0.00 | 52.3 | 100 |
| 0.10 | 0.10 | 51.3 | 98 |
| 0.25 | 0.25 | 45.3 | 87 |
| 0.50 | 0.50 | 25.6 | 49 |
| 0.75 | 0.75 | 10.8 | 21 |
| 1.00 | 1.00 | 7.2 | 14 |
| 1.50 | 1.50 | 6.0 | 12 |
| 2.00 | 2.00 | 5.8 | 11 |

| | | | |
|---|---|---|---|
| Tre: α,α-trehalose Glc: D-glucose | | | |

As is evident from the results in Table 4, the amount of acrylamide formed from equimolar L-asparagine and D-glucose was decreased as the increase of the amount of α,α-trehalose incorporated. In other words, α,α-trehalose suppressed the formation of acrylamide from L-asparagine and D-glucose by heating dose-dependently. In the case of using α,α-trehalose in a molar ratio of one or higher to L-asparagine or D-glucose, the saccharide gave almost a constant degree of suppression and suppressed the formation of acrylamide to less than 15%. It was revealed that the relative amount of acrylamide (%) was decreased less than 50% and 25% by using α,α-trehalose in molar ratios of 0.5 or higher and 0.75 or higher to L-asparagine or D-glucose under those conditions.

### Experiment 5

### Effect of α,α-trehalose on the formation of acrylamide from various amino acids and D-glucose

Effect of α,α-trehalose on the formation of acrylamide from various amino acids and D-glucose was investigated. Except for adding one mmol of D-glucose to a 20-ml tube with a ground glass stopper containing one mmol of either of various amino acids in Table 5, samples were treated with the same method as in Experiment 1 and determined for the amount of formed acrylamide, respectively. Further, samples prepared by adding one mmol of α,α-trehalose to the above reaction systems were also examined. The results are in Table 5. In the table, the acrylamide-formation(%) means the relative amount of acrylamide formed in the presence of α,α-trehalose, calculated by using the amount of acrylamide (mg) formed in the absence of α,α-trehalose as 100% for each sample.

**Table 5**

| Amino acid | | Formed acrylamide(mg) | | Acrylamide-formation (%) |
|---|---|---|---|---|
| | | Tre (-) * | Tre (+) ** | |
| Aliphatic Amino acid | Glycine | 21.6 | 11.4 | 53 |
| | h-Alanine | 8.9 | 4.1 | 46 |
| Branched Aliphatic Amino acid | L-Valine | 10.8 | 1.6 | 15 |
| | L-Leucine | 8.4 | 0.9 | 11 |
| | L-Isoleucine | 42.5 | 7.7 | 18 |
| Hydroxyl Amino acid | L-Serine | 10.4 | 4.2 | 40 |
| | L-Threonine | 15.0 | 7.7 | 51 |
| Acidic Amino acid | L-Aspartic acid | 7.7 | 1.0 | 13 |
| | L-Glutamic acid | 4.5 | 0.8 | 17 |
| Amide | L-Asparagine | 52.3 | 7.2 | 14 |
| | L-Glutamine | 6.5 | 1.0 | 15 |
| Basic Amino acid | L-Lysine | 5.1 | 2.1 | 40 |
| | L-Arginine | 4.9 | 1.9 | 39 |
| | L-Histidine | 0.0 | 0.0 | - |
| Sulfur containing amino acid | L-Cysteine | 1.4 | 1.0 | 72 |
| | L-Methionine | 1.8 | 1.2 | 67 |
| Aromatic Amino acid | L-Phenylalanine | 12.7 | 11.2 | 88 |
| | L-Tyrosine | 0.0 | 0.0 | - |
| | L-Tryptophan | 5.1 | 3.4 | 66 |
| Imino acid | L-Proline | 6.8 | 3.0 | 44 |

| | | | | |
|---|---|---|---|---|
| Tre: α,α-trehalose *: in the absence of α,α-trehalose **: in the presence of α,α-trehalose | | | | |

As shown in Table 5, the amount of acrylamide formed from amino acids and D-glucose by heating was varied depending on the kinds of amino acids. Relatively large amount of acrylamide was formed from two amino acids, L-asparagine and L-isoleucine. As is evident from the results in Table 5, it was revealed that α,α-trehalose remarkably suppressed the formation of acrylamide from L-isoleucine and D-glucose as in the case of L-asparagine. Although the amount of acrylamide formed was lesser than the cases of L-asparagine and L-isoleucine, the formation of acrylamide was observed in the cases of other amino acids, glycine, L-alanine, L-valine, L-leucine, L-serine, L-threonine, L-aspartic acid, L-glutamic acid, L-glutamine, L-lysine, L-arginine, L-cysteine, L-methionine, L-phenylalanine, L-tryptophan, and L-proline. Although the suppressing levels were varied depending on the kinds of amino acids, α,α-trehalose suppressed the formation of acrylamide. It has been known that L-valine, L-leucine, L-isoleucine, L-threonine, L-methionine, L-phenylalanine, and L-tryptophan are essential amino acids which can not be lacked for growth and keeping health of humans and animals.

In Experiments 1 to 5, the formation of acrylamide by heating was investigated using an aqueous-solution model, a reaction system under a relatively high moisture conditions. In the following Experiment 6 to 8, the formation of acrylamide by heating was investigated using a dry-model, a reaction system under a relatively low moisture condition, which is more closely related to the practical edible materials. In order to dissolve L-asparagine completely in the step of preparing samples, the amount of L-asparagine used for the test was reduced to 0.1 mmol. In the dry-model test, samples having various moisture contents were prepared by the steps of admixing 0.1 mmol L-asparagine, 0.1 mmol of D-glucose, and buffer with officinal potato starch as a base, making the mixtures into pastes, and drying arbitrarily. Successively, the effects of a saccharide on the formation of acrylamide in samples having various moisture contents were investigated by heating the pastes in a sealed tube.

### Experiment 6

### Effects of various non-reducing saccharides on the formation of acrylamide from L-asparagine and D-glucose by heating

Effects of various non-reducing saccharides on the formation of acrylamide from L-asparagine and D-glucose by heating were investigated by the dry-model tests. One ml of a saccharide solution (saccharide content: 0.1 mmol/ml) and 0.25 ml of 0.05 M sodium phosphate buffer (pH6.0) were added to a 20 ml-tube with a ground glass stopper containing one gram of officinal potato starch, 0.1 mmol (13 mg) of L-asparagine, and 0.1 mmol of D-glucose, and stirred to make the mixture into a paste having a moisture content of about 50%. To obtain the sample having a moisture content of about 10%, the sample was dried *in vacuo* at 60°C for two hours and then preserved in a desiccator controlled at a relative humidity of 22.4% for two days. To obtain a sample having a moisture content of about one %, the sample was dried *in vacuo* at 60°C for 16 hours. After measuring the moisture content of each sample, it was heated in an oil bath at 180°C for 20 minutes. The amount of acrylamide formed in the samples was measured by gas-chromatography. A sample (a paste, moisture content of about 50%) prepared with no drying treatment was also tested by the same procedure as above. To investigate the effects of non-reducing saccharides, six kinds of saccharides; α,α-trehalose, α,β-trehalose, sucrose, and D-mannitol, which showed a relatively strong ability of suppressing the formation of acrylamide in the aqueous solution-model in Experiment 2; and D-sorbitol and maltitol were used for the test. The moisture content of samples was measured using "MOISTURE BALANCE EB-330MOC", an electric moisture analyzer commercialized by Shimadzu Corporation, Kyoto, Japan. After heating, 10 ml of deionized water was added to each tube containing sample and the resulting mixture was stirred sufficiently. The amount of acrylamide in the samples was measured by gas chromatography as in the case of Experiment 1. a sample prepared with L-asparagine and D-glucose in the absence of other saccharide was used as a control. The results are in Table 6. In the table, each acrylamide-formation(%) means the relative amount of acrylamide formed in the presence of α,α-trehalose, calculated by using the amount of acrylamide (mg) formed in the absence of α,α-trehalose as

**Table 6**

| Saccharide | Moisture content of about 50% | | | Moisture content of about 10% | | | Moisture content of about 1% | | |
|---|---|---|---|---|---|---|---|---|---|
| | Measured moisture content (%) | Acrylamide | | Measured moisture content (%) | Acrylamide | | Measured moisture content (%) | Acrylamide | |
| | | Formed amount (mg) | Relative amount (%) | | Formed amount (mg) | Relative amount (%) | | Formed amount (mg) | Relative amount (%) |
| Control | 45.7 | 371.6 | 100 | 9.5 | 705.6 | 100 | 0.3 | 2708.5 | 100 |
| α,α-Trehalose | 46.3 | 46.9 | 13 | 8.9 | 189.3 | 27 | 0.4 | 1688.4 | 62 |
| α,β-Trehalose | 46.8 | 87.2 | 23 | 8.6 | 287.0 | 41 | 1.0 | 1693.1 | 63 |
| Sucrose | 46.3 | 419.2 | 113 | 9.2 | 898.8 | 127 | 0.7 | 3275.2 | 121 |
| D-Mannitol | 46.0 | 169.2 | 46 | 8.3 | 465.8 | 66 | 0.8 | 2255.8 | 83 |
| D-Sorbitol | 45.5 | 247.0 | 66 | 9.4 | 573.5 | 81 | 0.9 | 2544.0 | 94 |
| Maltitol | 45.4 | 224.5 | 60 | 8.3 | 516.6 | 73 | 0.7 | 2266.8 | 84 |

As is evident from the results in Table 6, it was confirmed that the formation of acrylamide from L-asparagine and D-glucose by heating was remarkably suppressed in the presence of α,α-trehalose, α,β-trehalose, and D-mannitol in any samples having moisture contents of about 5%, about 10%, and about 1% similarly with the results of Experiment 2. Specifically, effects of saccharides for suppressing the formation of acrylamide showed a tendency of decreasing by decreasing the moisture contents of samples to about 50%, about 10%, and about 1%. However, it was revealed that the formation of acrylamide can be suppressed by incorporating those saccharides into samples having moisture contents of about 10% or higher. While, in the case of using sucrose, the relative amount of acrylamide (%) was increased to 113-127% of that of control. It is considered that the degradation of sucrose was accelerated to form saccharides having strong ability of forming acrylamide by heating in a low moisture condition. D-Sorbitol and maltitol weakly suppressed the formation of acrylamide and the results were similar to that of Experiment 2.

### Experiment 7

### Effects of the moisture contents of samples, heating temperature, and α ,α-trehalose on the formation of acrylamide from L-asparagine and D-glucose

Effects of the moisture contents of samples, heating temperature, and the presence of α,α-trehalose as a saccharide having an ability of suppressing the formation of acrylamide on the formation of acrylamide from L-asparagine and D-glucose were further examined in detail. Except for preparing samples having various moisture contents by varying the periods for drying *in vacuo* at 60°C and using a heating temperature in three ways of 150°C, 180°C, and 200°C, samples were treated with same procedure in Experiment 6 and the amounts of formed acrylamide were measured. Samples treated in the absence of α,α-trehalose were used as controls. The results are in Table 7. The relative amount of formed acrylamide (%) was calculated with the amounts of formed acrylamide (mg) of controls at corresponding moisture contents as 100%.

**Table 7**

| | Measured moisture content (%) | Heating temperature | | | | | |
|---|---|---|---|---|---|---|---|
| | | 150°C | | 180°C | | 200°C | |
| | | Acrylamide | | Acrylamide | | Acrylamide | |
| | | Formed amount (mg) | Relative amount (%) | Formed amount (mg) | Relative amount (%) | Formed amount (mg) | Relative amount (%) |
| Control | 54.9 | 205.4 | 100 | 377.5 | 100 | 566.3 | 100 |
| | 43.6 | 209.4 | 100 | 404.1 | 100 | 582.3 | 100 |
| | 33.3 | 210.4 | 100 | 421.3 | 100 | 643.1 | 100 |
| | 22.5 | 219.3 | 100 | 507.4 | 100 | 702.1 | 100 |
| | 10.1 | 220.2 | 100 | 689.3 | 100 | 843.6 | 100 |
| | 5.8 | 230.2 | 100 | 1844.3 | 100 | 2133.7 | 100 |
| | 0.2 | 270.4 | 100 | 2566.3 | 100 | 3440.4 | 100 |
| α,α-Trehalose | 53.8 | 14.0 | 7 | 22.8 | 6 | 56.6 | 10 |
| | 44.9 | 26.7 | 13 | 48.2 | 12 | 75.7 | 13 |
| | 38.3 | 32.8 | 16 | 75.8 | 18 | 135.1 | 21 |
| | 25.1 | 59.7 | 27 | 111.6 | 22 | 168.5 | 24 |
| | 9.5 | 83.7 | 38 | 177.3 | 26 | 269.7 | 32 |
| | 5.4 | 143.7 | 62 | 940.6 | 51 | 1173.5 | 55 |
| | 0.1 | 207.7 | 77 | 1433.2 | 56 | 2752.3 | 80 |

As is evident from the results in Table 7, the formation of acrylamide was remarkably suppressed by allowing α,α-trehalose to coexist with L-asparagine and D-glucose at all heating conditions of 150°C, 180°C, and 200°C. Similarly with the results in Experiment 6, the effect of α,α-trehalose on the suppression of acrylamide formation tended to downward with decreasing the moisture contents of samples. However, in the case of being the moisture contents of samples about 10% or higher, it was revealed that the amount of formed acrylamide can be remarkably reduced to less than 30% of those of controls by incorporating equimolar α,α-trehalose into L-asparagine and D-glucose at all heating conditions of 150°C, 180°C, and 200°C.

### Experiment 8

### Effects of the moisture content of sample and the amount of α,α-trehalose on the formation of acrylamide from L-asparagine and D-glucose

Effects of the moisture content of sample and the amount of α,α-trehalose on the formation of acrylamide from L-asparagine and D-glucose were examined. Except for varying the amount of α,α-trehalose to 0.010, 0.025, 0.050, 0.075, 0.100, and 0.500 mmol, samples were treated with same procedure in Experiment 6 and the amounts of formed acrylamide were measured. Samples treated in the absence of α,α-trehalose were used as controls. The results are in Table 8. Relative amount of formed acrylamide (%) was calculated with the amounts of formed acrylamide (mg) of controls at corresponding moisture contents as 100%.

**Table 8**

| Amount of α,α-Trehalose (mmol) | Moisture content of about 50% | | | Moisture content of about 10% | | | Moisture content of about 1% | | |
|---|---|---|---|---|---|---|---|---|---|
| | Measured moisture content (%) | Acrylamide | | Measured moisture content (%) | Acrylamide | | Measured moisture content (%) | Acrylamide | |
| | | Formed amount (mg) | Relative amount (%) | | Formed amount (mg) | Relative amount (%) | | Formed amount (mg) | Relative amount (%) |
| 0.000 | 53.3 | 373.5 | 100 | 9.5 | 677.7 | 100 | 0.6 | 2494.9 | 100 |
| 0.010 | 54.1 | 137.9 | 37 | 9.3 | 543.7 | 80 | 0.5 | 1978.4 | 79 |
| 0.025 | 53.8 | 105.0 | 28 | 10.1 | 460.0 | 68 | 0.6 | 1804.8 | 72 |
| 0.050 | 53.5 | 47.0 | 13 | 9.8 | 363.9 | 54 | 0.5 | 1535.6 | 62 |
| 0.075 | 54.3 | 44.6 | 12 | 10.1 | 290.5 | 43 | 0.5 | 1479.1 | 59 |
| 0.100 | 53.4 | 24.0 | 6 | 10.4 | 165.0 | 24 | 0.6 | 1362.1 | 55 |
| 0.500 | 54.5 | 10.2 | 3 | 10.8 | 113.4 | 17 | 0.7 | 1306.0 | 52 |

As is evident from the results in Table 8, the formation of acrylamide from L-asparagine and D-glucose was suppressed with increase of the amount of α,α-trehalose. The present dry-model test revealed that the relative amount of formed acrylamide (%) can be suppressed, in the case of samples having a moisture content of about one %, to less than 65%, and in the case of samples having a moisture content of about 10%, to less than 55% by incorporating α,α-trehalose in a molar ratio of 0.5 or higher into L-asparagine or D-glucose.

From the results of dry-model tests described in the above Experiments 6 to 8, followings were revealed:
(1) The formation of acrylamide from L-asparagine and D-glucose by heating can be remarkably suppressed by incorporating α,α-trehalose, α,β-trehalose or D-mannitol into a sample even in the case of having a low moisture content.
(2) In the case of being the moisture content of sample about 10% or higher, the formation of acrylamide can be remarkably suppressed by incorporating equimolar of α,α-trehalose into L-asparagine and D-glucose.
(3) The relative amount of acrylamide (%) can be suppressed to less than 65% by incorporating a,a-trehalose in a molar ratio of 0.5 or higher into L-asparagine or D-glucose.

From the results in Experiment 1, it was revealed that L-ascorbic acid has a higher ability of forming acrylamide than D-glucose. Therefore, L-ascorbic acid was used as a saccharide having an ability of forming acrylamide in following Experiment 9 to 12. In those experiments, the amount of L-asparagine was reduced to 0.1 mmol to dissolve it completely. Further, in Experiment 10 to 12, the heating temperature was set to 100°C in consideration for cooking edible materials containing 1-ascorbic acid such as fruits and vegetables.

### Experiment 9

### Effects of various saccharides on the formation of acrylamide from L-asparagine and L-ascorbic acid

Effects of various saccharides on the formation of acrylamide from L-asparagine and L-ascorbic acid by heating were examined. One ml of mixture (containing 0.1 mmol of L-ascorbic acid) , prepared by mixing 0.1 M L-ascorbic acid aqueous solution and 0.5 M sodium L-ascorbate aqueous solution to give a pH of 4.0, was put into a 20 ml-tube with ground glass stopper, containing 0.1 mmol of L-asparagine, and then 0.1 mmol of any saccharide shown in Table 9 was admixed with the mixture. But in the cases of α-maltotriosyl α,α-trehalose, α-cyclodextrin and β-cyclodextrin, 200 mg each (corresponding to about 0.17 to 0.24 mmol) of saccharide was used. Except for heating at 150°C for 20 min, samples were treated with the same procedure in Experiment 1 and the amounts of formed acrylamide were determined. A sample, containing L-asparagine and L-ascorbic acid, without admixing any other saccharide was examined as a control. The results are in Table 9. Relative amount of acrylamide (%) is a relative value calculated using the amount of acrylamide (mg) in control sample as 100%.

**Table 9**

| Saccharide | Acrylamide | |
|---|---|---|
| | Formed amount (mg) | Relative amount (%) |
| Control (L-Asn* + AsA**) | 129.2 | 100 |
| D-Glucose | 155.6 | 120 |
| D-Fructose | 229.6 | 178 |
| D-Sorbitol | 90.1 | 70 |
| D-Mannitol | 52.3 | 40 |
| D-Erythritol | 51.1 | 40 |
| D-Xylitol | 96.3 | 74 |
| D-Galactose | 170.0 | 132 |
| L-Arabinose | 128.1 | 99 |
| α,α-Trehalose | 11.5 | 9 |
| α,β-Trehalose | 18.9 | 15 |
| Kojibiose | 64.1 | 50 |
| Nigerose | 111.0 | 86 |
| Maltose | 161.4 | 125 |
| Isomaltose | 77.9 | 60 |
| Maltitol | 96.9 | 75 |
| Isomaltitol | 75.3 | 58 |
| Sucrose | 160.1 | 124 |
| Palatinose | 116.2 | 90 |
| Reduced Palatinose | 54.0 | 42 |
| Lactose | 119.1 | 92 |
| Lactitol | 111.4 | 86 |
| Maltotriose | 134.7 | 104 |
| α-Glucosyl α,α-trehalose | 55.2 | 43 |
| Maltotetraose | 119.2 | 92 |
| α-Maltosyl α,α-trehalose | 57.8 | 45 |
| Cyclic tetrasaccharide*** | 115.8 | 90 |
| α-Maltotriosyl α,α-trehalose | 93.1 | 72 |
| α-Cyclodextrin | 105.3 | 81 |
| β-Cyclodextrin | 47.5 | 37 |

| | | |
|---|---|---|
| *: L-Asparagine **: L-Ascorbic acid ***: Cyclic tetrasaccharide constructed by 4 glucose molecules bound by alternating α-1,6 and α-1,3 glucosidic linkages. | | |

As is evident from the results in Table 9, it was revealed that the formation of acrylamide from L-asparagine and L-ascorbic acid by heating was remarkably suppressed in the presence of several saccharides similarly with the case of using D-glucose as a saccharide having an ability of forming acrylamide in Experiment 2. Particularly, α,α-trehalose, α,β-trehalose, D-mannitol, D-erythritol, reduced palatinose, α-glucosyl α,α-trehalose, α-maltosyl α,α-trehalose, and α-cyclodextrin suppressed the relative amount of formed acrylamide (%) to less than 50%. More particularly, α,α-trehalose and α,β-trehalose remarkably suppressed the relative amount of formed acrylamide (%) to less than 16%. In this experiment, sucrose did not suppress and promoted the formation of acrylamide, and the result was opposite one in Experiment 2. The reason of this was presumed that sucrose was hydrolyzed into D-glucose and D-fructose under the heating condition and the resultants reactedwithL-asparagine to form acrylamide. From the results of the present experiment and Experiment 2, it is evident that α-maltosyl α,α-trehalose is a saccharide having an ability of suppressing the formation of acrylamide as well as α,α-trehalose and α-glucosyl α,α-trehalose.

### Experiment 10

### Effects of reaction pH and α,α-trehalose on the formation of acrylamide from L-asparagine and L-ascorbic acid

Effects of reaction pH and α,α-trehalose on the formation of acrylamide from L-asparagine and L-ascorbic acid by heating were examined. One ml of mixture (containing 0.1 mmol of L-ascorbic acid), prepared by mixing 0.1 M L-ascorbic acid aqueous solution and 0.5 M sodium L-ascorbate aqueous solution to give a pH of 3, 4, 5, 6, or 6.5 was put into a 20 ml-tube with ground glass stopper, containing 0.1 mmol of L-asparagine. Except for heating at 100°C for 20 min, samples were treated with the same procedure in Experiment 1 and the amounts of formed acrylamide were determined. Further, samples prepared by admixing 0.1 mmol of α,α-trehalose with above corresponding samples were also examined. The results are in Table 10. In the table, relative amount of acrylamide (%) in the presence of α,α-trehalose is a relative value calculated using the amount of acrylamide (mg) in corresponding samples in the absence of α,α-trehalose as 100%.

**Table 10**

| pH | In the absence of Tre | | | In the presence of Tre | | | |
|---|---|---|---|---|---|---|---|
| | Initial pH | pH after heating | Formed acrylamide (mg) | Initial pH | pH after heating | Formed acrylamide (mg) | Relative amount (%) |
| 3 | 3.0 | 3.6 | 0.23 | 3.0 | 3.1 | 0.07 | 30 |
| 4 | 4.0 | 4.1 | 0.52 | 4.1 | 4.1 | 0.14 | 26 |
| 5 | 5.1 | 5.2 | 0.61 | 5.1 | 5.1 | 0.20 | 33 |
| 6 | 6.0 | 6.0 | 1.65 | 6.1 | 6.0 | 0.22 | 13 |
| 6.5 | 6.5 | 6.0 | 2.05 | 6.6 | 6.4 | 0.45 | 22 |

| | | | | | | | |
|---|---|---|---|---|---|---|---|
| Tre: α,α-trehalose | | | | | | | |

As shown in Table 10, pH values of samples were only slightly changed before and after of heating in both cases of the presence and absence of α,α-trehalose. It was revealed that acrylamide was formed even in the case of heating at 100°C though the amount of formed acrylamide is low because of small amounts of samples and a low heating temperature. As is evident from the results in Table 10, in the absence of α,α-trehalose, the amount of acrylamide increased with increase of reaction pH and suddenly increased at pH 6 and 6.5. In the presence of α,α-trehalose, the amounts of acrylamide in samples at any reaction pHs were remarkably lower than those in the absence of α,α-trehalose. Therefore, it was confirmed that the formation of acrylamide can be remarkably suppressed by incorporating α,α-trehalose into samples.

### Experiment 11

### Effects of the amount of α,α-trehalose on the formation of acrylamide from L-asparagine and L-ascorbic acid

The dose dependency of α,α-trehalose on the ability of suppressing the formation of acrylamide from L-asparagine and D-glucose by heating was examined. One ml of the mixture (containing 0.1 mmol of L-ascorbic acid) prepared by mixing L-ascorbic acid solution and sodium L-ascorbate solution to give a pH of 6 was put into a 20 ml-tube with ground glass stopper, containing 0.1 mmol of L-asparagine. Except for adding 0.000, 0.010, 0.025, 0.050, 0.075, 0.100, 0.150, 0.250, or 0.500 mmol of α,α-trehalose, each sample was heated by the same procedure and the amount of formed acrylamide was determined with the same procedure in Experiment 1. The results are in Table 11. The relative amount of acrylamide (%) was calculated using the amount of acrylamide (mg) formed in the absence of α,α-trehalose as 100%.

**Table 11**

| Amount of α,α-trehalose (mmol) | Molar ratio (Tre/AsA) | Acrylamide | |
|---|---|---|---|
| | | Formed amount (mg) | Relative amount (%) |
| 0.000 | 0.00 | 1.65 | 100 |
| 0.010 | 0.10 | 1.59 | 96 |
| 0.025 | 0.25 | 1.50 | 91 |
| 0.050 | 0.50 | 1.24 | 75 |
| 0.075 | 0.75 | 0.78 | 47 |
| 0.100 | 1.00 | 0.22 | 13 |
| 0.150 | 1.50 | 0.21 | 13 |
| 0.250 | 2.50 | 0.18 | 11 |
| 0.500 | 5.00 | 0.16 | 10 |

| | | | |
|---|---|---|---|
| Tre: α,α-trehalose AsA: L-ascorbic acid | | | |

As is evident from the results in Table 11, the amount of acrylamide formed from L-asparagine and L-ascorbic acid was decreased with increase of the amount of α,α-trehalose incorporated. The formation of acrylamide from L-asparagine and L-ascorbic acid was suppressed by α,α-trehalose dose-dependently. The degrees of the suppression were almost constant, i.e., the relative amounts of acrylamide (%) were less than 15%, when α,α-trehalose was incorporated in a molar ratio of one or higher to L-asparagine or L-ascorbic acid. Under the present condition, it was revealed that the relative amount of acrylamide (%) was reduced to 75% and less than 50% by incorporating α,α-trehalose in molar ratios of 0.5 or higher and 0.75 or higher to L-asparagine or L-ascorbic acid, respectively.

### Experiment 12

### Effects of pH and α,α-trehalose on the formation of acrylamide from L-asparagine and derivatives of L-ascorbic acid

Except for using "AA2G®", L-ascorbic acid 2-glucoside commercialized by Hayashibara Biochemical Laboratories Inc., Okayama, Japan, and "Stay-C 50®", L-ascorbicacid 2-phosphate tri-sodium salt commercialized by Roche Vitamins Japan K.K., Tokyo, Japan, as derivatives of L-ascorbic acid instead of L-ascorbic acid, and a heating temperature in two ways of 100°C and 125°C; effects of pH and the amount of α,α-trehalose on the formation of acrylamide from L-asparagine and derivatives of L-ascorbic acid were examined with the same procedure in Experiment 10. Samples prepared by using L-ascorbic acid instead of derivatives of L-ascorbic acid were tested by the same procedure as a control test. The results obtained by using L-ascorbic acid, L-ascorbic acid 2-glucoside, and L-ascorbic acid 2-phosphate are in Table 12, Table 13, and Table 14, respectively. In the tables, relative acrylamide-formation (%) mean relative amount of acrylamide in the presence of α,α-trehalose, calculated with that in the absence of α,α-trehalose as 100% at corresponding pHs.

**Table 12**

| pH | Treatment at 100°C | | | | |
|---|---|---|---|---|---|
| | Tre (-)* | | Tre (+)** | | |
| | pH after heating | Formed Acrylamide (mg) | pH after heating | Formed Acrylamide (mg) | Relative acrylamide-Formation (%) |
| N.A. | - | - | - | - | - |
| 3 | 3.6 | 0.23 | 3.1 | 0.07 | 30 |
| 4 | 4.1 | 0.53 | 4.1 | 0.14 | 26 |
| 5 | 5.2 | 0.61 | 5.1 | 0.20 | 33 |
| 6 | 6.0 | 1.65 | 6.0 | 0.22 | 13 |
| 6.5 | 6.0 | 2.05 | 6.4 | 0.45 | 22 |

| pH | Treatment at 125°C | | | | |
|---|---|---|---|---|---|
| | Tre (-)* | | Tre (+)** | | |
| | pH after heating | Formed Acrylamide (mg) | pH after heating | Formed Acrylamide (mg) | Relative acrylamide-Formation (%) |
| N.A. | - | - | - | - | - |
| 3 | 3.5 | 0.70 | 3.1 | 0.06 | 9 |
| 4 | 4.1 | 0.85 | 4.1 | 0.08 | 9 |
| 5 | 5.2 | 1.85 | 5.1 | 0.13 | 7 |
| 6 | 6.0 | 10.75 | 6.0 | 1.67 | 16 |
| 6.5 | 6.2 | 15.07 | 6.4 | 1.84 | 12 |

| | | | | | |
|---|---|---|---|---|---|
| Tre: α,α-trehalose *: in the absence of α,α-trehalose **: in the presence of α,α-trehalose N.A.: Not adjusted. -: Not tested. | | | | | |

**Table 13**

| pH | Treatment at 100°C | | | | |
|---|---|---|---|---|---|
| | Tre (-)* | | Tre (+)** | | |
| | pH after heating | Formed acrylamide (mg) | pH after heating | Formed acrylamide (mg) | Relative acrylamide-Formation (%) |
| N.A. | 2.06 | 0.03 | 2.07 | 0.01 | 33 |
| 3 | 3.02 | 0.02 | 3.10 | 0.00 | 0 |
| 4 | 4.11 | 0.01 | 4.13 | 0.00 | 0 |
| 5 | 5.05 | 0.00 | 5.01 | 0.00 | - |
| 6 | 6.01 | 0.00 | 6.06 | 0.00 | - |
| 6.5 | 6.52 | 0.00 | 6.53 | 0.00 | - |

| pH | Treatment at 125°C | | | | |
|---|---|---|---|---|---|
| | Tre (-)* | | Tre (+)** | | |
| | pH after heating | Formed acrylamide (mg) | pH after heating | Formed acrylamide (mg) | Relative acrylamide-Formation (%) |
| N.A. | 2.03 | 0.20 | 2.01 | 0.06 | 30 |
| 3 | 3.01 | 0.39 | 3.11 | 0.06 | 15 |
| 4 | 4.13 | 0.08 | 4.06 | 0.01 | 13 |
| 5 | 5.06 | 0.05 | 5.08 | 0.00 | 0 |
| 6 | 6.02 | 0.00 | 6.03 | 0.00 | - |
| 6.5 | 6.55 | 0.00 | 6.54 | 0.00 | - |

| | | | | | |
|---|---|---|---|---|---|
| Tre: α,α-trehalose *: in the absence of α,α-trehalose **: in the presence of α,α-trehalose N.A.: Not adjusted. | | | | | |

**Table 14**

| pH | Treatment at 100°C | | | | |
|---|---|---|---|---|---|
| | Tre (-)* | | Tre (+)** | | |
| | pH after heating | Formed acrylamide (mg) | pH after heating | Formed acrylamide (mg) | Relative acrylamide-Formation (%) |
| N.A. | 9.47 | 0.09 | 9.46 | 0.02 | 22 |
| 3 | 3.08 | 0.22 | 3.04 | 0.01 | 5 |
| 4 | 4.01 | 0.03 | 4.03 | 0.00 | 0 |
| 5 | 4.99 | 0.01 | 5.00 | 0.00 | 0 |
| 6 | 6.02 | 0.05 | 6.06 | 0.01 | 20 |
| 6.5 | 6.90 | 0.07 | 6.91 | 0.02 | 29 |

| pH | Treatment at 125°C | | | | |
|---|---|---|---|---|---|
| | Tre (-)* | | Tre (+)** | | |
| | pH after heating | Formed acrylamide (mg) | pH after heating | Formed acrylamide (mg) | Relative acrylamide-Formation (%) |
| N.A. | 9.48 | 14.20 | 9.47 | 1.33 | 9 |
| 3 | 3.07 | 9.30 | 3.01 | 0.83 | 9 |
| 4 | 4.03 | 0.49 | 4.06 | 0.01 | 2 |
| 5 | 5.00 | 0.13 | 5.09 | 0.02 | 15 |
| 6 | 6.02 | 3.40 | 6.04 | 0.53 | 16 |
| 6.5 | 6.91 | 13.10 | 6.93 | 1.66 | 13 |

| | | | | | |
|---|---|---|---|---|---|
| Tre: α,α-trehalose *: in the absence of α,α-trehalose **: in the presence of α,α-trehalose N.A.: Not adjusted. | | | | | |

As is evident from the results in Table 12, in the case of heating at 100°C, the results were same with those of Experiment 10. In the case of heating at 125°C, the amount of acrylamide in the absence of α,α-trehalose increased with increase of reaction pH, and suddenly increased at pH 6 and at pH 6.5. In the presence of α,α-trehalose, the amounts of acrylamide were remarkably lower than those in the absence of α,α-trehalose at any reaction pHs. While, as is evident from the results in Table 13, L-ascorbic acid 2-glucoside, a derivative of L-ascorbic acid, was stable campared with L-ascorbic acid, and hardly formed acrylamide in the ranges of pH 4 to 6, and pH 5 to 6.5 by heating at 100°C and 125°C, respectively. Although acrylamide was formed at both heating temperature at lower pH conditions, the amounts of acrylamide were low. The reason of forming acrylamide from L-ascorbic acid 2-glucoside at lowpH conditions was presumed that L-ascorbic acid 2-glucoside was partially hydrolyzed to form L-ascorbic acid and D-glucose by heating under acidic conditions and acrylamide was formed from the resulting L-ascorbic acid and/or D-glucose and L-asparagine. Since the amount of acrylamide formed in the presence of α,α-trehalose was remarkably lower than that in the absence of α,α-trehalose, it was confirmed even in this case that a remarkable suppressing effect can be obtained by incorporating α,α-trehalose into samples. As is evident from the results in Table 14, in the case of L-ascorbic acid 2-phosphate, the amount of acrylamide formed by heating at 100°C was low in the pH range of 4 to 7 in comparison with the case of L-ascorbic acid. However, acrylamide was formed from L-ascorbic acid 2-phosphate in the same level with L-ascorbic acid at pHs of lower than 4. In the case of heating at 125°C, although the amount of acrylamide formed from L-ascorbic acid 2-phosphate was lower than that from L-ascorbic acid in a pH range of 4 to 6, however, it was 10-folds or more than that from L-ascorbic acid under the condition of pH of lower than 4 and was almost equal amount with that from L-ascorbic acid under the condition of pH of higher than 6. Compared the results with those obtained by using L-ascorbic acid 2-glucoside in Table 12, L-ascorbic acid 2-glucoside was superior to L-ascorbic acid 2-phosphate because the amount of acrylamide formed was low in a relatively wider pH range and at higher heating temperatures. Also in the case of using L-ascorbic acid 2-phosphate, the formation of acrylamide was remarkably suppressed in the presence of α,α-trehalose.

### Experiment 13

### Effects of various phenol substances on the formation of acrylamide from L-asparagine and D-glucose

Effects of various phenol substances on the formation of acrylamide from L-asparagine and D-glucose by heating were investigated.
0.25 mg of any one of 22 kinds of phenol substances shown in Table 15 was put into a 20 ml-tube with ground glass stopper, containing 0.1 mmol (13.2 mg) of L-asparagine and 0.1 mmol (1.80 mg) of D-glucose. Except for adding one ml of 0.05 M sodium phosphate buffer (pH6.0), samples were treated with the procedure in Experiment 1 and the amounts of formed acrylamide were determined. A sample, containing L-asparagine and D-glucose, without adding any phenol substances was examined as a control. The results are in Table 15. Relative amount of acrylamide (%) is a relative value calculated using the amount of acrylamide in control sample as 100%.

As for the phenolic compounds used in the frame of the invention, the reader is referred to the claims.

**Table 15**

| Phenol compound | Amount* (µmol) (0.25 mg) | Molar ratio (to Glc or Asn) | Acrylamide | |
|---|---|---|---|---|
| | | | Formed amount (mg) | Relative amount (%) |
| Control | - | - | 152.33 | 100 |
| Catechin | 0.86 | 0.0086 | 111.98 | 74 |
| Epicatechin | 0.86 | 0.0086 | 101.49 | 67 |
| Epigalocatechin | 0.82 | 0.0082 | 87.02 | 57 |
| Quercetin | 0.83 | 0.0083 | 84.51 | 55 |
| Rutin | 0.41 | 0.0041 | 68.77 | 45 |
| Naringin | 0.43 | 0.0043 | 61.22 | 40 |
| Hesperidin | 0.41 | 0.0041 | 83.91 | 55 |
| Flavanon | 1.12 | 0.0112 | 166.91 | 110 |
| Kaempferol | 0.87 | 0.0087 | 141.48 | 93 |
| Galangin | 0.93 | 0.0093 | 162.32 | 107 |
| Cinnamic acid | 1.69 | 0.0169 | 55.67 | 37 |
| Quinic acid | 1.30 | 0.0130 | 79.33 | 52 |
| 3,4-Dihydro-cinnamic acid | 1.39 | 0.0139 | 107.27 | 70 |
| 3-Coumaric acid | 1.78 | 0.0178 | 84.91 | 56 |
| 4-Coumaric acid | 1.78 | 0.0178 | 81.24 | 53 |
| *p*-Nitrophenol | 1.80 | 0.0180 | 67.14 | 44 |
| Trypthanthrin | 1.01 | 0.0101 | 155.28 | 102 |
| Curcumin | 0.68 | 0.0068 | 66.00 | 43 |
| Scopoletin | 1.30 | 0.0130 | 89.06 | 58 |
| *p*-Hydroxybenzoic acid n-propyl | 1.39 | 0.0139 | 134.77 | 88 |
| Coumarin | 1-71 | 0.0171 | 237.92 | 156 |
| Protoanthocyanidin | ** | ** | 74.95 | 49 |

| | | | | |
|---|---|---|---|---|
| Glc: D-glucose Asn: L-asparagine *: Amount is calculated as anhydrate. **: Not calculated as mole because the compound is a polymer. | | | | |

As is evident from the results in Table 15, it was revealed that the formation of acrylamide from L-asparagine and D-glucose by heating was remarkably suppressed in the presence of some phenol substances. Specifically, it was confirmed that the formation of acrylamide from L-asparagine and D-glucose was suppressed in the presence of phenol substances except for galangin, flavanon, trypthanthrin, and coumarin, i.e., catechin, epicatechin, epigalocatechin, quercetin, rutin, naringin, hesperidin, kaempferol, cinnamic acid, quinic acid, 3,4-dihydro-cinnamic acid, 3-coumaric acid, 4-coumaric acid, *p*-nitrophenol, curcumin, scopoletin, p-hydroxybenzoic acid n-propyl, and protoanthocyanidin in a molar ratios of 0.004 or higher to L-asparagine or D-glucose. Especially, itwas confirmed that rutin, naringin, and hesperidin remarkably suppressed the formation of acrylamide in a level of less than 60% by incorporating them relatively low amount, in a molar ratio of about 0.004 to L-asparagine and D-glucose.

### Experiment 14

### Effect of α,α-trehalose on the formation of acrylamide in fried potatoes during the cooking

The effect of α,α-trehalose on the formation of acrylamide during the cooking of food materials was examined on fried potatoes as an example.

### Experiment 14-1

### Preparation of fried potatoes

To 30 grams of water, 0, 0.02, 0.06, 0.13, 0.27, 0.53, and 1.3 grams of "TREHA®", a high purity hydrous α,α-trehalose commercialized by Hayashibara Shoji Inc., Okayama, Japan, were added respectively, and dissolved by heating up to about 80°C for 30 seconds using a microwave oven. Successively, 20 grams each of "QUEEN'S CHEF MASHED POTATOES", a dried mashed potatoes commercialized by Miki Shokuhin Co. Ltd., Hyogo, Japan, was admixed with above each aqueous solution, mixed to homogeneity, and shaped into a disk having a diameter of 22 mm and a thickness of 3 mm (The amounts of α,α-trehalose to the dried mashed potatoes were 0, 0.1, 0.3, 0.6, 1.2, 2.4, and 6.0% (w/w), respectively.). The resulting shaped products were fried with edible oil at 150°C for 4 minutes using "MODEL MACH F3", a electric fryer commercialized by Mach Co. Ltd., Tokyo, Japan, to make into fried potatoes. It was revealed that dried mashed potatoes, used in the present experiment, contained 7.2% (w/w) of moisture, and about 8% (w/w) of saccharides having an ability of forming acrylamide (3% (w/w) of D-glucose, 2.8% (w/w) of D-fructose, and 2.1% (w/w) of sucrose), by the measurement by the present inventors.

### Experiment 14-2

### Measurement of acrylamide formed in fried potatoes

Five grams each of fried potatoes, obtained in Experiment 14-1, was admixed with 25 grams of deionized water and then the resulting mixture was stood for 10 minutes. Successively, the mixture was homogenized for three minutes using "MODEL ULTRA-TU-PRAX", a homogenizer commercialized by IKA Japan K.K., Nara, Japan. The resulting homogenate was centrifuged at 4, 000 rpm for 60 minutes. The resulting supernatant was frozen at -80°C, and then thawed. The thawed solution was centrifuged to obtain supernatant. Successively, two milliliters of the resulting supernatant (sample) was admixed with 0.2 ml of 2, 3, 3-d3-acrylamide standard solution (2 ppm), which is used as an internal standard, applied to "OASIS HBL 500 mg CARTRIDGE", a solid phase extraction column commercialized by Nihon Waters K.K., Tokyo, Japan, which is pre-conditioned with five ml of methanol and 5 ml of deionized water, and eluted two ml of deionized water for 10 times to separate total 10 fractions. Among the fractions, a fraction containing acrylamide was filtrated and used as a sample for analyzing acrylamide. In this experiment, in order to measure the amount of acrylamide, it was measured with a liquid chromatography/mass spectrometry (LC/MS).

LC/MS was carried out using "FINNIGAN ION-TRAP LC/MS/MS SYSTEM", an LC/MS equipment commercialized by Thermo Electron K.K., Tokyo, Japan, with following conditions.

### (Conditions of LC/MS)

### <LC>

Column: Hydrosphere C18 HS-3C2 (I.D.; 2 mm, Length; 150 mm, Particle Size; 5 µm), commercialized by YMC Co. Ltd., Kyoto, Japan
Eluent: methanol:water (5:95)
Flow rate: 0.1 ml/min, Column temperature: 40°C
Time for analysis: 12 min, Sample injection volume: 20 to 40 µl

### <MS>

Ionization: electro-spray (ESI)
Conditions for ionization:
   Flow rate of sheath gas: 5 (arb), Flow rate of ox gas: 0 (arb),
   Spray voltage: 5 (kV), Capillary voltage: 6 (kV),
   Capillary temperature: 200°C, Tube lens off-set voltage: 25 (V),
   Time for analysis: 10 min
   (Samples eluted at 4 to 7 min in LC were introduce to ion source)
Detection: Single ion monitoring (SIM)

Ions having a m/z of 71.5 to 72.6 and those having m/z of 74.5 to 75.6 were measured as acrylamide and 2,3,3-d3-acrylamide (internal standard), respectively.

The ionization efficiencies of acrylamide and 2,3,3-d3-acrylamide, internal standard, were examined in advance using those standard preparations. Measurement of acrylamide was carried out for three times per sample. The concentration value of the internal standard which is admixed with sample was multiplied by the ratio of peak area of acrylamide to that of 2,3,3-d3-acrylamide. The average value obtained by three independent measurements was multiplied by the above ionization efficiency to calculate the amount of acrylamide in the sample. The results of the measurements are in Table 13. The amount of acrylamide formed in fried potatoes was represented by ppm, the concentration of acrylamide per fried potato products.

**Table 16**

| α,α-Trehalose content (to dried mashed potatoes, w/w %) | Formed acrylamide (per fried potato product) | | Relative amount of acrylamide (%) |
|---|---|---|---|
| | ppm* | SD** | |
| 0.0 (control) | 0.71 | 0.04 | 100 |
| 0.1 | 0.67 | 0.09 | 94 |
| 0.3 | 0.57 | 0.06 | 80 |
| 0.6 | 0.45 | 0.03 | 63 |
| 1.2 | 0.23 | 0.03 | 32 |
| 2.4 | <0.01 | - | <2 |
| 6.0 | <0.01 | - | <2 |

| | | | |
|---|---|---|---|
| *: Average value of three independent measurements. **: Standard deviation | | | |

As shown in Table 16, 0.71 ppm of acrylamide was formed in fried potatoes cooked without admixing α,α-trehalose with dried mashed potatoes. On the other hand, in the case of fried potatoes prepared by admixing α,α-trehalose with mashed potatoes, the amount of acrylamide formed in fried potatoes was decreased with increase of the amount of α,α-trehalose. For example, the amount of acrylamide was decreased to 0.45 ppm (about 63% of control) in the fried potatoes prepared by admixing 0.6% (w/w) α,α-trehalose with mashed potatoes. In the case of fried potatoes prepared by admixing 2.4% (w/w) or more α,α-trehalose with mashed potatoes, the amount of acrylamide formed in fried potatoes was less than 0.01 ppm, i.e., less than detection limit. It was revealed that the formation of acrylamide during the cooking of fried potatoes was remarkably suppressed by the addition of α,α-trehalose. In this experiment, α,α-trehalose remarkably suppresses the formation of acrylamide with a relatively low amount, 0.6 to 2.4% (w/w) in the presence of about 8% (w/w) of saccharides having an ability of forming acrylamide, contained in dried mashed potatoes. From the result, it is suggested that the formation of acrylamide during the cooking of fried potatoes depends on not only the amount of saccharides having an ability of forming acrylamide, contained in dried mashed potatoes, but also the amount of organic substances having an amino group (s). Also, it is suggested that the later especially controls the formation of acrylamide.

From the results in Experiment 1 to 12, it is revealed that the formation of acrylamide can be remarkably suppressed by incorporating a saccharide selected from the group consisting of α,α-trehalose, α,β-trehalose, reduced palatinose, α-glucosyl α,α-trehalose, α-maltosyl α,α-trehalose,D-mannitol,D-erythritol, and β-cyclodextrin, specifically, α,α-trehalose or α,β-trehalose, into edible materials when heating an edible material containing organic substances having an amino group(s) and saccharides having an ability of forming acrylamide by reacting with them. Further, from the result of Experiment 13, it is revealed that the formation of acrylamide can be remarkably suppressed by incorporating specific phenol substances, specifically, rutin, naringin, or hesperidin, into such edible materials. Furthermore, from the result in Experiment 14, it is confirmed that the amount of acrylamide formed in cooked foods can be remarkably decreased by incorporating α,α-trehalose into food materials when practical food materials are cooked by heating.

Following examples concretely explain the present invention.

### Example 1

### Potato chips

After washing "MAY QUEEN", potatoes produced in Hokkaido Prefecture, Japan, with water, they were peeled and washed again, and then, sliced up in a 1.5 mm in thickness using "V-slicer MV-50", a professional-quality slicer commercialized by Shinkousya Co., Ltd., Saitama, Japan. The resulting sliced potatoes were immersed in water, dehydrated, and then soaked into two-folds by weight of 20% (w/w) α,α-trehalose aqueous solution produced by dissolving "TREHA®", a high purity hydrous crystalline α,α-trehalose commercialized by Hayashibara Shoji Inc., Okayama, Japan, at 80°C for two hours for penetrating α,α-trehalose into potatoes. Successively, after removing the saccharide solution, the sliced potatoes were fried using "MACH F-3", an electric fryer commercialized by Mach Co., Ltd., Tokyo, Japan, whose oil temperature was controlled at 180°C, for two minutes to prepare potato chips. Edible mixed oil (mixture of edible colza oil and edible soybean oil) was used for flying.

The potato chops have a satisfactory shape and taste. Since the potato chips comprise α,α-trehalose, the formation of acrylamide by frying treatment is suppressed. Therefore, the potato chips have a satisfactory safety and can be advantageously used as a snack.

### Example 2

### Fried potatoes

After washing "MAY QUEEN", potatoes produced in Hokkaido Prefecture, Japan, with water, they were cut into a square pole-shape having a one cm in thickness using a cutter. The resulting potato fragments were soaked in an aqueous solution with 0.1% sodium chloride, 20% (w/w) α,α-trehalose ("TREHA®", aα, α-trehalose product commercialized by Hayashibara Shoji Inc., Okayama, Japan) at 50°C for 20 minutes. After dehydrating and blanching for three minutes, the potatoes were frozen for preserving. The resulting frozen potatoes were fried by a conventional method to produce fried potatoes.

The fried potatoes have a satisfactory texture and taste. Since the fried potatoes comprise α,α-trehalose, the formation of acrylamide by frying treatment is suppressed. Therefore, the fried potatoes have a satisfactory safety and can be advantageously used intact as a snack or side dishes.

### Example 3

### Crackers

Seventy parts by weight of medium flour, 0.25 part by weight of yeast and 30 parts by weight of water were mixed and kneaded to produce a sponge dough. Successively, 30 parts by weight of soft flour, 10 parts by weight of shortening, 1.5 parts by weight of starch syrup containing D-glucose, 1.5 parts by weight of sodium chloride, 0.65 part by weight of sodium bicarbonate, and 1.5 parts by weight of "TREHA®", a high purity hydrous crystalline α,α-trehalose commercialized by Hayashibara Shoji Inc., Okayama, Japan, were admixed with a sponge dough and further kneaded. After shaping the resulting dough into crackers by a conventional method, the soda crackers were prepared by baking them in an oven at 180°C for 15 minutes.

The crackers have a satisfactory taste and palatable. Since the crackers comprise α,α-trehalose, the formation of acrylamide by baking is suppressed. Therefore, the crackers can be advantageously used intact as a snack.

### Example 4

### Butter rolls (buns)

One hundred and eighteen parts by weight of hard flour, eight parts by weight of sucrose, 10 parts by weight of "TREHA®", a high purity hydrous crystalline α,α-trehalose commercialized by Hayashibara Shoji Inc., Okayama, Japan, 1.7 parts by weight of sodium chloride, two parts by weight of skim milk, two parts by weight of "KAIYO KOBO", a yeast commercialized by Sankyo Foods Co., Ltd., Tokyo, Japan, 12 parts by weight of egg, 10 parts by weight of a salt-free butter, 20 parts by weight of milk, and 75 parts by weight of water were kneaded by a kneader at 24°C for six minutes at a low speed and five minutes at a moderate speed. After stopping the kneading for a while, the mixture was further kneaded for 4.5 minutes at a moderate speed to make into doughs. Successively, the dough was fermented for 50 minutes as "floor time". After dividing the dough into 40 grams each, and which were then rounded, and fermented for 20 minutes as "bench time". Then, the doughs were further fermented for 50 minutes using a proofing room controlled at 40°C and a humidity of 80%. After the fermentation, the doughs were baked for seven minutes in an oven which the upper and lower temperatures were controlled to 230°C and 200°C, respectively, to produce butter rolls.

The butter rolls show a fluffy bulge, satisfactory color, and good taste. Since the butter rolls comprise α,α-trehalose, the formation of acrylamide by baking is suppressed. Therefore, the butter rolls have a satisfactory safety.

### Example 5

### Roasted sliced almonds

Commercially available sliced almond (1mm in thickness) was soaked in 50% (w/w) α,β-trehalose solution prepared by dissolving "NEOTREHALOSE", an α,β-trehalose product commercialized by Hayashibara Biochemical Laboratories Inc., Okayama, Japan, at 70°C for 15 minutes. After dehydrating, the resulting sliced almond was roasted using an electric oven at 180°C to make into a roasted sliced almond.

The roasted sliced almond has a satisfactory taste. Since the roasted sliced almond comprises α,β-trehalose, the formation of acrylamide by roasting is suppressed. Therefore, the roasted sliced almond can be advantageously used intact as a snack or material for various confectioneries and bakery products.

### Example 6

### Roasted wheat germ

After thawing 78 parts by weight of frozen raw wheat germ, 20 parts by weight of "TREHA®", a hydrous crystalline α,α-trehalose commercialized by Hayashibara Shoji Inc., Okayama, Japan, five parts by weight of a powdery α-glycosyl α,α-trehalose (comprising 4.1% of α-glucosyl β,α-trehalose, 52.5% of α-maltosyl α,α-trehalose on a dry solid basis), prepared by the method of Example A-2 disclosed in Japanese Patent Kokai No. 143,876/95, were admixed with the raw wheat germ and then two parts by weight of 5% (w/w) pullulan aqueous solution prepared by dissolving "PULLULAN PF-20", a pullulan product commercialized by Hayashibara Shoji Inc., Okayama, Japan, was added to the mixture. The resulting mixture was placed in a sealed-type extruder, heated, kneaded, and extruded into granules, and then the resulting granules were dried. Further, the resulting granules were roasted by a conventional method to prepare a roasted wheat germ.

The roasted wheat germ has a satisfactory taste. Since the roasted wheat germ comprise α,α-trehalose, the formation of acrylamide by roasting is suppressed. Therefore, the roasted wheat germ has a satisfactory safety. Furthermore, the product can be used intact as a healthy food or material for various foods and beverages because the product is rich in minerals such as selenium, zinc, etc., vitamins and dietary fiber.

### Example 7

### Coating for "tempura"

After dissolving 20 grams of "TREHA®", an α,α-trehalose commercialized by Hayashibara Shoji Inc., Okayama, Japan, in 380 ml of cold water, the resulting solution was admixed with scrambled commercially available raw egg and about 230 grams of sifted soft flour, and then stirred gently to prepare a coating for *"tempura"*.

Since the product comprises α,α-trehalose, the formation of acrylamide is suppressed by using the product when various ingredients for *"tempura"* such as meat, vegetables, etc., are fried to make *"tempura"*.

### Example 8

### Powdery mashed potatoes

One hundred parts by weight of "QUEEN'S CHEF MASHED POTATOES", commercially available dried mashed potatoes commercialized by Miki Shokuhin Co. Ltd., Hyogo, Japan, and 2.5 parts by weight of "TREHA®", a high purity hydrous crystalline α,α-trehalose powder commercialized by Hayashibara Shoji Inc., Okayama, Japan, were mixed to homogeneity to produce powdery mashed potatoes.

Since the product comprises α,α-trehalose, the formation of acrylamide can be suppressed when the product is fried together with ingredients such as meat, vegetable, etc. for cooking potato croquettes. Therefore, the mashed potatoes are composition having a satisfactory safety and the ability of suppressing the formation of acrylamide.

### Example 9

### Potato croquettes

One hundred parts by weight of powdery mashed potatoes, obtained in Experiment 8, were seasoned with salt and pepper and then admixed with 100 parts by weight of milk and 200 parts by weight of hot water. The resulting mixture was stood for 10 minutes to reconstitute the mashed potatoes. Successively, 100 parts by weight of minced meat and 100 parts by weight of minced onion were fried up with butter, seasoned with salt and pepper, and admixed with the above reconstituted mashed potatoes for shaping into croquettes. According to conventional method, the resultants were sequencially placed into flour, scrambled raw egg, and crumbs, and fried up in edible oil at 180°C to prepare potato croquettes. Since the croquettes are prepared by powdery mashed potatoes containing α,α-trehalose, the formation of acrylamide by frying is suppressed. Therefore, the potato croquettes have a satisfactory safety.

### Example 10

### Sweet potato snack

One hundred parts by weight of wheat flour, 300 parts by weight of ground raw sweet potato, and 30 parts by weight of water were mixed. Successively, 20 parts by weight of "TREHA®", a high purity hydrous crystalline α,α-trehalose commercialized by Hayashibara Shoji Inc., Okayama, Japan, and 0.2 part by weight of "αG-RUTIN H", glycosyl-transferred rutin commercialized by Hayashibara Shoji Inc., Okayama, Japan, were admixed with the above mixture and the resulting mixture was steamed at 100°C for 10 minutes to prepare a dough. After extending the dough to give one mm in thickness, the extended dough was hardened by preserving at 10°C for 12 hours. The resulting dough was cut into preferable shapes to obtain a dough for sweet potato snack. Successively, a sweet potato snack was prepared by frying the dough in vegetable oil at 170-180°C. Since the snack comprises α,α-trehalose and glycosyl-transferred rutin, the formation of acrylamide by frying is suppressed. Therefore, the sweet potato snack has a satisfactory safety.

### Example 11

### Crisp pretzels

One hundred parts by weight of soft flour, 0.8 part by weight of baking powder, one part by weight of sodium chloride, and 1.5 parts by weight of "TREHA®", a powdery high purity hydrous crystalline α,α-trehalose commercialized by Hayashibara Shoji Inc., Okayama, Japan, were mixed. Successively, 35 parts by weight of milk, 13 parts by weight of salad oil, 12 parts by weight of grated cheese, and 4.5 parts by weight of minced parsley were admixed with the above mixture and kneaded into a dough. After extending the resulting dough to give two mm in thickness, the extended dough was cut to give five mm in width to obtain a dough for pretzels. Successively, crisp pretzels were prepared by baking the dough in an oven controlled at 230-260°C for six minutes. Since the pretzels comprise α,α-trehalose, the formation of acrylamide by baking is suppressed. Therefore, the crisp pretzels have a satisfactory safety.

### Example 12

### Seasoned instant noodles

98.5 parts by weight of wheat flour (hard flour), 1.5 parts by weight of "TREHA®", a powdery high purity hydrous crystalline α,α-trehalose commercialized by Hayashibara Shoji Inc., Okayama, Japan, 0.01 part by weight of "αG-RUTIN H", a glycosyl-transferred rutin commercialized by Hayashibara Shoji Inc., Okayama, Japan, were admixed with 30 parts by weight of 0.5% salt water, and then the resulting mixture was kneaded by conventional method to make into noodles having 0.9 mm in thickness, and steamed for 75 seconds in a steamer. Successively, 30 parts by weight of a chicken soup was admixed with the steamed noodles and allowed to absorb the soup, and then fried in a salad oil at 145°C for 80 seconds to make into seasoned instant noodles. Since the instant noodles comprise α,α-trehalose and glycosyl-transferred rutin, the formation of acrylamide by frying is suppressed. Therefore, the seasoned instant noodles have a satisfactory safety.

### Example 13

### Composition comprising L-ascorbic acid

A composition comprising L-ascorbic acid is obtained by mixing one part by weight of powdery L-ascorbic acid and nine parts by weight of "TREHA®", powdery high purity hydrous crystalline α,α-trehalose commercialized by Hayashibara Shoji Inc., Okayama, Japan, to homogeneity and granulating.

Since the product comprises α,α-trehalose together with L-ascorbic acid, it can be used for suppressing the formation of acrylamide effectively, even in the case of producing other composition requiring L-ascorbic acid by heating with an edible material having a possibility of forming acrylamide by reacting with L-ascorbic acid. The product is in a granule form having a satisfactory fluidity (without solidification), handleability, and stability during the preservation. Therefore, the product can be advantageously used for producing various compositions having a high quality and satisfactory safety, which contains L-ascorbic acid, such as foods, beverages, cosmetics, pharmaceuticals, and intermediates thereof.

### Example 14

### Composition comprising an L-ascorbic acid derivative

A powdery composition comprising L-ascorbic acid derivative is obtained by mixing one part by weight of "AA2G®", a powdery L-ascorbic acid 2-glucoside commercialized by Hayashibara Biochemical Laboratories Inc., Okayama, Japan, and nine parts by weight of "TREHA®", a powdery high purity hydrous crystalline α, α-trehalose commercialized by Hayashibara Shoji Inc., Okayama, Japan, to homogeneity.

Since the product comprises α,α-trehalose together with L-ascorbic acid 2-glucoside, it can be used for suppressing the formation of acrylamide effectively, even in the case of producing other composition requiring L-ascorbic acid by heating with an edible material having a possibility of forming acrylamide by reacting with L-ascorbic acid. The product is in a granule form having a satisfactory fluidity (without solidification), handleability, and stability during the preservation. Therefore, the product can be advantageously used for producing various compositions having a high quality and satisfactory safety, which contains L-ascorbic acid derivative, such as foods, beverages, cosmetics, pharmaceuticals, and intermediates thereof.

### Example 15

### Composition comprising L-ascorbic acid

A composition comprising L-ascorbic acid is obtained by mixing one part by weight of powdery L-ascorbic acid, five parts by weight of "TREHA®", a powdery high purity hydrous crystalline α,α-trehalose commercialized by Hayashibara Shoji Inc., Okayama, Japan, and 0.02 part by weight of "αG-RUTIN", glycosyl-transferred rutin commercialized by Hayashibara Shoji Inc., Okayama, Japan, to homogeneity and granulating.

Since the product comprises α,α-trehalose and glycosyl-transferred rutin together with L-ascorbic acid, it can be used for suppressing the formation of acrylamide effectively, even in the case of producing other composition requiring L-ascorbic acid by heating with an edible material having a possibility of forming acrylamide by reacting with L-ascorbic acid. The product is granule having a satisfactory fluidity (without solidification), handleability, and stability during the preservation. Therefore, the product can be advantageously used for producing various compositions having a high quality and satisfactory safety, which contains L-ascorbic acid, such as foods, beverages, cosmetics, pharmaceuticals, and intermediates thereof.

### Example 16

### W/O type emollient cream

The following ingredients were mixed and dissolved by heating according to the following proportion.

| | |
|---|---|
| Bee wax | 3.0 parts by weight |
| Cetanol | 5.0 parts by weight |
| Reduced lanolin | 5.0 parts by weight |
| Squalane | 31.5 parts by weight |
| Self-emulsifying glycerin monostearate | 4.0 parts by weight |
| Lipophilic glycerin monostearate | 2.0 parts by weight |
| Sorbitan monolanolinate ester | 2.0 parts by weight |

Separately, the following ingredients were mixed according to the following proportion and dissolved by heating at 80°C.

| | |
|---|---|
| Preservative | 0.2 part by weight |
| Sodium L-glutamate | 3.2 parts by weight |
| L-Serine | 0.8 part by weight |
| "TREHA®", α,α-trehalose commercialized by Hayashibara Shoji Inc., Okayama, Japan, | 2.0 parts by weight |
| "NEOTREHALOSE", α,β-trehalose commercialized by Hayashibara Biochemical Laboratories Inc., Okayama, Japan, | 4.0 parts by weight |
| "AA2G®", L-ascorbic acid 2-glucoside commercialized by Hayashibara Biochemical Laboratories Inc., Okayama, Japan, | 1.0 part by weight |
| Propylene glycol | 5.0 parts by weight |
| Purified water | 30.5 parts by weight |

The former mixture was admixed gradually with the later mixture with stirring and cooled to 30°C or lower to produce a W/O type emollient cream.

Since the product comprises α,α-trehalose and α,β-trehalose together with amino acids and L-ascorbic acid 2-glucoside, the formation of acrylamide by heating is suppressed. Further, the emollient cream has a satisfactory moisture-retaining property, extending property, and gloss, and is preferable as a cosmetic having a satisfactory safety.

### Example 17

### Nutritious supplement drink

Four parts by weight of L-isoleucine, six parts by weight of L-leucine, eight parts by weight of L-lysine hydrochloride salt, eight parts by weight of L-phenylalanine, one part by weight of L-tyrosine, eight parts by weight of tryptophan, eight parts by weight of L-valine, one part by weight of L-aspartic acid, one part by weight of L-serine, one part by weight of L-methionine, two parts by weight of L-threonine, and eight parts by weight of L-arginine hydrochloride salt were mixed to prepare a mixture of amino acids. Successively, four parts by weight of the mixture of amino acids, one part by weight of "AA2G®", a powdery L-ascorbic acid 2-glucoside commercialized by Hyashibara Biochemical Laboratories Inc., Okayama, Japan, six parts by weight of "TREHA®", a powdery high purity hydrous crystalline α,α-trehalose commercialized by Hayashibara Shoji Inc., Okayama, Japan, two parts by weight of "NEOTREHALOSE", a α,β-trehalose commercialized by Hyashibara Biochemical Laboratories Inc., Okayama, Japan, 0.02 part by weight of "αG-HESPERIDINE PA", a glycosyl-transferred hesperidine commercialized by Hayashibara Shoji Inc., Okayama, Japan, 0.01 part by weight of thiamin nitrate, 0.04 part by weight of nicotinamide, and 86.05 parts by weight of water were mixed to homogeneity. The resulting mixture was bottled in a 50 ml-brown bottle, sealed, and sterilized by heating at 85°C for 30 minutes to make into a nutritional supplement drink.

Since the product comprises α,α-trehalose and α,β-trehalose together with amino acids and L-ascorbic acid 2-glucoside, the formation of acrylamide by heating is suppressed. Therefore, the product is preferable as a nutritional supplement drink containing amino acids, having a satisfactory safety.

### INDUSTRIAL APPLICABILITY

Compositions, having a satisfactory safety and less possibility of causing cancer and nerve disorder, can be produced by applying the method for suppressing the formation of acrylamide of the present invention to produce various compositions such as foods, beverages, cosmetics, pharmaceuticals, and intermediate products thereof. Compositions having an ability of suppressing the formation of acrylamide of the present invention, which comprise an organic substance(s) having an amino group(s) and/or saccharide(s) having an ability of forming acrylamide and an organic substance (s) having an ability of suppressing the formation of acrylamide, can be advantageously used for producing foods, beverages, cosmetics, pharmaceuticals, and intermediates thereof as agricultural-, stockbreeding-, marine-products, those processed products, seasonings, or sweeteners. Specifically, in the case of using L-ascorbic acids as a saccharide having an ability of forming acrylamide, the formation of acrylamide can be effectively suppressed by using the composition as a composition, comprising L-ascorbic acids, having an ability of suppressing the formation of acrylamide, for producing foods, beverages, cosmetics, pharmaceuticals, and intermediates thereof, which comprise an ingredient having a possibility of forming acrylamide by heating, and requiring L-ascorbic acids. The composition comprising L-ascorbic acids has a satisfactory safety and can be advantageously used for producing various compositions, which have a high quality and L-ascorbic acid (vitamin C) is supplemented, such as foods, beverages, cosmetics, pharmaceuticals, and intermediates thereof.

The present invention has a remarkable effectiveness described above and is a significantly important invention that greatly contributes to the field which produces or uses foods, beverages, cosmetics, pharmaceuticals, and intermediate products thereof.

## Claims

1. A method for suppressing the formation of acrylamide, comprising the steps of:
incorporating a saccharide and/or phenol, having an ability of suppressing the formation of acrylamide, into an edible material having a possibility of forming acrylamide by heating; and
heating the resulting mixture;
said saccharide having an ability of suppressing the formation of acrylamide is one or more saccharides selected from the group consisting of α,α-trehaloge, α,β-trehalose, reduced palatinose, α-glucosyl α,α-trehalose, α-maltosyl α,α-trehalose, D-mannitol, D-erythritol, and β-cyclodextrin; and said phenol having an ability of suppressing the formation of acrylamide is one or more phenols selected from the group consisting of cinnamic acid, quinic acid, 3,4-dihydro-cinnamic acid, 3-coumaric acid, 4-coumaric acid, *p*-nitrophenol, curcumin, scopoletin, p-hydroxybenzoic acid n-propyl, and saccharide derivatives thereof.

2. The method of claim 1, wherein said edible material comprises an organic substance having an amino group(s) and a saccharide having an ability of forming acrylamide by reacting with the organic substance.

3. The method of claim 1 or 2, wherein said edible material is selected from the group consisting of agricultural, stockbreeding- and marine-products, and processed products thereof.

4. The method of claim 2 or 3, wherein said organic substance having an amino group (s) is an amino acid or its salt selected from the group consisting of glycine, L-alanine, L-valine, L-leucine, L-isoleucine, L-serine, L-threonine, L-aspartic acid, L-glutamic acid, L-asparagine, L-glutamine, L-lysine, L-arginine, L-cysteine, L-methionine, L-phenylalanine, L-tryptophan, and L-proline; and peptides and proteins having those amino acids as components.

5. The method of claim 2, wherein said saccharide having an ability of forming acrylamide by reacting with the organic substance is one or more saccharides selected from the group consisting of:
a monosaccharide selected from the group consisting of D-xylose, L-arabinose, D-glucose, D-fructose and D-galactose;
a reducing oligosaccharide having as a component one or more monosaccharides selected from the group consisting of D-xylose, L-arabinose, D-glucose, D-fructose and D-galactose;
a non-reducing oligosaccharide having as a component D-fructose; and
L-ascorbic acid and the like selected from the group consisting of L-ascorbic acid, L-ascorbic acid derivative, and a salt thereof.

6. The method of claim 5, wherein said L-ascorbic acid derivative is 2-o-α-D-glucosyl-L-ascorbic acid (L-ascorbic acid 2-glucoside), L-ascorbic acid 2-phosphate, or a salt thereof.

7. The method of claim 2, wherein said saccharide having an ability of suppressing the formation of acrylamide is incorporated into either an organic substance having an amino group (s) or a saccharide having an ability of forming acrylamide in said edible material in a molar ratio of 0.5 or higher to said organic substance or said saccharide having an ability of forming acrylamide.

8. The method of claim 2, wherein said phenol having an ability of suppressing the formation of acrylamide is incorporated into either an organic substance having an amino group(s) or a saccharide having an ability of forming acrylamide in said edible material in a molar ratio of 0.004 or higher to said organic substance or said saccharide having an ability of forming acrylamide.

9. The method of claim 1, wherein said heating is a treatment for heating at a temperature of 80°C or higher, selected from the group consisting of baking, frying, roasting, heating with microwave oven, heating in retort pouch, heating by pressurizing, boiling, steaming, simmering, and heating for sterilization.

## Patentansprüche

1. Verfahren zur Unterdrückung der Bildung von Acrylamid, umfassend die Schritte:
Einlagern eines Saccharids und/oder Phenols, das die Fähigkeit aufweist, die Bildung von Acrylamid zu unterdrücken, in einen essbaren Stoff, der die Möglichkeit besitzt, beim Erwärmen Acrylamid zu bilden; und
Erwärmen der erhaltenen Mischung;
wobei das Saccharid, das die Fähigkeit besitzt, die Bildung von Acrylamid zu unterdrücken, ausgewählt ist aus einem oder mehreren Sacchariden aus der Gruppe bestehend aus α,α-Trehalose, α,β-Trehalose, reduzierte Palatinose, α-Glucosyl-α,α-Trehalose, α-Maltosyl-α,α-Trehalose, D-Mannitol, D-Erythritol und β-Cyclodextrin; und das Phenol, das die Fähigkkeit besitzt, die Bildung von Acrylamid zu unterdrücken, ausgewählt ist aus einem oder mehreren Phenol(en) aus der Gruppe bestehend aus Zimtsäure, Chinasäure, 3,4-Dihydro-Zimtsäure, 3-Cumarsäure, 4-Cumarsäure, p-Nitrophenol, Curcumin, Scopoletin, p-Hydroxybenzoesäure-n-propyl und Saccharidderivate davon.

2. Verfahren nach Anspruch 1, wobei der essbare Stoff eine eine Aminogruppe(n) aufweisende organische Substanz und ein die Fähigkeit zur Bildung von Acrylamid durch Reaktion mit der organischen Substanz aufweisendes Saccharid umfasst.

3. Verfahren nach Anspruch 1 oder 2, wobei der essbare Stoff ausgewählt ist aus der Gruppe bestehend aus Ackerbau-, Viehzucht- und Meeresprodukten sowie daraus hergestellten Produkten.

4. Verfahren nach Anspruch 2 oder 3, wobei die eine Aminogruppe(n) aufweisende organische Substanz eine Aminosäure oder ihr Salz ist, ausgewählt aus der Gruppe bestehend aus Glycin, L-Alanin, L-Valin, L-Leucin, L-Isoleucin, L-Serin, L-Threonin, L-Asparaginsäure, L-Glutaminsäure, L-Asparagin, L-Glutamin, L-Lysin, L-Arginin, L-Cystein, L-Methionin, L-Phenylalanin, L-Typtophan und L-Prolin; sowie Peptide und Proteine, die diese Aminiosäuren als Bestandteile aufweisen.

5. Verfahren nach Anspruch 2, wobei das die Fähigkeit zur Bildung von Acrylamid durch Reaktion mit der organischen Substanz aufweisende Saccharid ausgewählt ist aus einem oder mehreren Sacchariden aus der Gruppe bestehend aus:
einem Monosaccharid ausgewählt aus der Gruppe D-Xylose, L-Arabinose, D-Glucose, D-Fructose und D-Galactose;
einem reduzierenden Oligosaccharid, das als Bestandteil ein oder mehrere Monosaccharide aufweist, ausgewählt aus der Gruppe bestehend aus D-Xylose, L-Arabinose, D-Glucose, D-Fructose und D-Galactose;
einem nichtreduzierenden Oligosaccharid, das als Bestandteil D-Fructose aufweist; und
L-Ascorbinsäure und ähnliche ausgewählt aus der Gruppe bestehend aus L-Ascorbinsäure, L-Ascorbinsäurederivat und Salze davon.

6. Verfahren nach Anspruch 5, wobei das L-Ascorbinsäurederivat 2-o-α-D-Glucosyl-L-Ascorbinsäure (L-Ascorbinsäure-2-glucosid), L-Ascorbinsäure-2-phosphat oder ein Salz davon ist.

7. Verfahren nach Anspruch 2, wobei das die Fähigkeit zur Unterdrückung der Bildung von Acrylamid aufweisende Saccharid entweder in eine eine Aminogruppe(n) aufweisende organische Substanz oder in ein die Fähigkeit zur Bildung von Acrylamid aufweisendes Saccharid in dem essbaren Stoff in einem molaren Verhältnis von 0.5 oder mehr in Bezug auf die organische Substanz oder das die Fähigkeit zur Bildung von Acrylamid aufweisende Saccharid eingelagert wird.

8. Verfahren nach Anspruch 2, wobei das die Fähigkeit zur Unterdrückung der Bildung von Acrylamid aufweisende Phenol entweder in eine eine Aminogruppe(n) aufweisende organische Substanz oder in ein die Fähigkeit zur Bildung von Acrylamid aufweisendes Saccharid in dem essbaren Stoff in einem molaren Verhältnis von 0.5 oder mehr in Bezug auf die organische Substanz oder das die Fähigkeit zur Bildung von Acrylamid aufweisende Saccharid eingelagert wird.

9. Verfahren nach Anspruch 1, wobei die Erwärmung eine Wärmebehandlung bei einer Temperatur von 80 °C oder mehr ist, ausgewählt aus der Gruppe bestehend aus Backen, Braten, Rösten, Erwärmen in einem Mikrowellenofen, Erwärmen in einem Retortenofen, Erwärmen unter Druck, Kochen, Dämpfen, Sieden und Erhitzen zur Sterilisation.

## Revendications

1. Procédé pour supprimer la formation d'acrylamide, comprenant les étapes consistant :
à incorporer un saccharide et/ou du phénol, ayant une aptitude à supprimer la formation d'acrylamide, à une substance comestible présentant une possibilité de formation d'acrylamide par chauffage ; et
à chauffer le mélange résultant ;
ledit saccharide présentant une aptitude à supprimer la formation d'acrylamide consistant en un ou plusieurs saccharides choisis dans le groupe consistant en l'α,α-tréhalose, l'α,β-tréhalose, le palatinose réduit, l'α-glucosyle-α,α-tréhalose, l'α-maltosyle-α,α-tréhalose, le D-mannitol, le D-érythritol et la β-cyclodextrine ; et ledit phénol présentant une aptitude à supprimer la formation d'acrylamide consistant en un ou plusieurs phénols choisis dans le groupe consistant en l'acide cinnamique, l'acide quinique, l'acide 3,4-dihydro-cinnamique, l'acide 3-coumarique, l'acide 4-coumarique, le p-nitrophénol, la curcumine, la scopolétine, l'acide p-hydroxybenzoïque, l'acide n-propyle et leurs dérivés saccharidiques.

2. Procédé suivant la revendication 1, dans lequel ladite substance comestible comprend une substance organique ayant un ou plusieurs groupes amino et un saccharide présentant une aptitude à former de l'acrylamide par réaction avec la substance organique.

3. Procédé suivant la revendication 1 ou 2, dans lequel ladite substance comestible est choisie dans le groupe consistant en des produits agricoles, des produits d'élevage du bétail et des produits marins, ainsi que leurs produits obtenus par transformation.

4. Procédé suivant la revendication 2 ou 3, dans lequel ladite substance organique ayant un ou plusieurs groupes amino est un aminoacide ou son sel choisi dans le groupe consistant en la glycine, la L-alanine, la L-valine, la L-leucine, la L-isoleucine, la L-sérine, la L-thréonine, l'acide L-aspartique, l'acide L-glutamique, la L-asparagine, la L-glutamine, la L-lysine, la L-arginine, la L-cystéine, la L-méthionine, la L-phénylalanine, la L-tryptophane et la L-proline ; et des peptides et protéines comprenant ces aminoacides comme constituants.

5. Procédé suivant la revendication 2, dans lequel ledit saccharide présentant une aptitude à former de l'acrylamide par réaction avec la substance organique consiste en un ou plusieurs saccharides choisis dans le groupe consistant en :
un monosaccharide choisi dans le groupe consistant en le D-xylose, la L-arabinose, le D-glucose, le D-fructose et le D-galactose ;
un oligosaccharide réducteur comprenant comme constituant un ou plusieurs monosaccharides choisis dans le groupe consistant en le D-xylose, la L-arabinose, le D-glucose, le D-fructose et le D-galactose ;
un oligosaccharide non-réducteur comprenant comme constituant le D-fructose ; et
l'acide L-ascorbique ou un composé similaire choisi dans le groupe consistant en l'acide L-ascorbique, un dérivé d'acide L-ascorbique et un de leurs sels.

6. Procédé suivant la revendication 5, dans lequel ledit dérivé d'acide L-ascorbique et l'acide 2-O-α-D-glucosyl-L-ascorbique (2-glucoside d'acide L-ascorbique), le 2-phosphate d'acide L-ascorbique ou un de leurs sels.

7. Procédé suivant la revendication 2, dans lequel ledit saccharide présentant une aptitude à supprimer la formation d'acrylamide est incorporé à une substance organique comprenant un ou plusieurs groupes amino ou un saccharide présentant une aptitude à former de l'acrylamide dans ladite substance comestible en un rapport molaire égal ou supérieur à 0,5 par rapport à ladite substance organique ou audit saccharide présentant une aptitude à former de l'acrylamide.

8. Procédé suivant la revendication 2, dans lequel ledit phénol présentant une aptitude à supprimer la formation d'acrylamide est incorporé à une substance organique comprenant un ou plusieurs groupes amino ou à un saccharide présentant une aptitude à former de l'acrylamide dans ladite substance comestible en un rapport molaire égal ou supérieur à 0,004 par rapport à ladite substance organique ou audit saccharide présentant une aptitude à former de l'acrylamide.

9. Procédé suivant la revendication 1, dans lequel ledit chauffage est un traitement de chauffage à une température égale ou supérieure à 80°C, choisi dans le groupe consistant en la cuisson au four, la cuisson en friture, le rôtissage, le chauffage au moyen d'un four à micro-ondes, le chauffage dans une poche d'autoclavage, le chauffage par mise sous pression, l'ébullition, la cuisson à la vapeur d'eau, la cuisson à feu doux et le chauffage à des fins de stérilisation.
